(19) Europäisches Patentamt

European Patent Office

Office européen des brevets

(11) Publication number: **0 409 858 B1**

## EUROPEAN PATENT SPECIFICATION

(45) Date of publication of patent specification: **08.12.93**

(51) Int. Cl.5: **C07K 13/00**, A61K 37/02, C12N 15/00

(21) Application number: **89904014.1**

(22) Date of filing: **17.03.89**

(86) International application number:
**PCT/DK89/00059**

(87) International publication number:
**WO 89/08666 (21.09.89 89/23)**

The file contains technical information submitted after the application was filed and not included in this specification

(54) **HEPARIN-BINDING PROTEINS, DNA CODING FOR THEM, PROCESSES FOR PRODUCING THEM AS WELL AS THERAPEUTIC PREPARATIONS CONTAINING THEM.**

(30) Priority: **17.03.88 DK 1453/88**
**17.02.89 DK 737/89**

(43) Date of publication of application:
**30.01.91 Bulletin 91/05**

(45) Publication of the grant of the patent:
**08.12.93 Bulletin 93/49**

(84) Designated Contracting States:
**AT BE CH DE FR GB IT LI LU NL SE**

(56) References cited:
**EP-A- 241 136**
**EP-A- 298 723**
**WO-A-87/07183**
**WO-A-88/10269**

**DIALOG INFORMATION SERVICES, file 55, BIOSIS 81-89, no. 85050038; H. RAUVALA et al.&NUM;**

(73) Proprietor: **NOVO NORDISK A/S**
**Novo Allé**
**DK-2880 Bagsvaerd(DK)**

(72) Inventor: **FLODGAARD, Hans**
**Melvillevej 6**
**DK-2900 Hellerup(DK)**
Inventor: **OSTERGAARD, Erik**
**Borglumvej 19B 1.tv.**
**DK-2720 Vanlose(DK)**
Inventor: **THOMSEN, Johannes**
**Fasanvaenget 506**
**DK-2980 Kokkedal(DK)**
Inventor: **BAYNE, Stephen**
**Pilevej 16**
**Tune**
**DK-4000 Roskilde(DK)**

**DIALOG INFORMATION SERVICES, file 55, BIOSIS 81-89, no. 83082612; H. PANDE et al.&NUM;**

**DIALOG INFORMATION SERVICES, file 55, BIOSIS 81-89, no. 79028069; G.J. RAUGI et al.&NUM;**

(74) Representative: **Sundien, Gunnar et al**
**HOFMAN-BANG & BOUTARD A/S**
**Adelgade 15**
**DK-1304 Copenhagen K (DK)**

## Description

The present invention concerns heretofore unknown heparin-binding proteins or equivalent modifications hereof which release angiogenesis and improved granulation tissue formation in wounds in animal models. The invention also relates to processes for producing the proteins and pharmaceutical preparations containing the proteins suitable for stimulation of tissue repair in man, in particular for topical application to external wounds. The proteins are furthermore characterized by in the normal condition being glycoproteins.

Normal tissue repair follows an orderly sequence of cellular and biochemical events, which is initiated by injury and results in new tissue formation.

Resting fibroblasts at the wound edge divide, migrate towards the avascular wound space, and produce collagen. New capillaries bud from preexisting venules and capillaries and migrate towards the wound edge. These processes continue until the edge of the healing wound fuses, filling the wound space with a vascularized collagen fibroblast mesh (granulation tissue). Finally, epithel cells divide and cover the granulation tissue and the repair process is finished.

The blood platelets are the first cellular element of importance for the healing of an acute wound within the first 24 hours. Then the healing processes are taken over by neutrophilic granulocytes followed by macrohages and lymphocytes, which can all be seen to migrate into the wound in an orderly sequence during the next 2 to 3 days after the tissue damage. It is these inflammatory cells which eventually ensure correct healing via carefully adapted release of paracrinic growth factors.

In recent years some understanding has accumulated concerning the mechanism for the action for these growth factors, which are called Platelet derived growth factor (PDGF), Transforming growth factor alpha (TGF$\alpha$), and Transforming growth factor $\beta$ (TGF$\beta$) in the wound healing process. PDGF is initially released from the $\alpha$-granules of the blood platelets when the platelets adhere to the edges of the fresh wound and have strong chemotaxis for fibroblasts (Grotendorst, G.R. et al. (1981). Proc. Natl. Acad. Sci. USA. 78: 3669-3672) in addition to being mitogenic to the same cells in the presence of either TGF$\alpha$ or epidermal growth factor (EGF) (Deuel, T.F. et al. (1985) Cancer Surv. 4: 633-653.

PDGF activates fibroblasts to release collagenase (Bauer, E.A. et al. (1985) Proc. Natl. Acad. Sci. USA, 82: 4132-4136) and thus contributes to remodelling the matrix, an essential element in the wound healing.

TGF$\beta$ is found in relatively high concentrations in the blood platelets and is also shown to be released from v granules during the clot formation. This growth factor plays an important part in the matrix formation in wounds and has also been found to have a regulatory influence on a variety of other growth factors, such as PDGF, EGF, and TGF$\alpha$.

TGF$\beta$ exhibits strong chemotactic activity for monocytes. Thus the growth factors initially released from platelets immediately after wounding also play an important role by stimulating migration of inflammatory cells to the wound. TGF$\beta$ may recruit monocytes from the circulation and subsequently activate them to the secretory phenotype (Wiseman, D.M. et al. (1988) Biochemical and Biophysical Research Communications 157, 793-800).

Once the monocytes have obtained this phenotype and are now better named macrophages, they can be shown to secrete the same factors found in platelets plus several others of great importance to the repair process.

Thus monocytes/macrophages excrete growth regulatory factors, such as Platelet derived growth factor (PDGF), Transforming growth factor beta (TGF$\beta$), Transforming growth factor alpha (TGF$\alpha$), Basic fibroblast growth factor (BFGF). Insulin-like growth factor one (IGF-1) Bombesin, Granulocyte-stimulating factor (GSF), Granulocyte-macrophage colony stimulating factor (GM-CSF), Monocyte stimulating factor (M-CSF) and Interleukin-1 (IL-1). The secretory products also include proteases, complement proteins, monocyte-derived neutrophil-activating factor, arachidonates and Tumor necrosis factor alpha (TNF$\alpha$). (Rom, W.N. et al. (1988) J. Clin. Invest. 82, 1685-1693), Rappolee D.A. et al (1988) Science 241, 708-712), (for review see Unanue, E.R. et al (1987). Science 236, 551-557).

All these macrophage derived paracrinic growth factors participate in the healing process, but many details concerning the mechanism and the complex interaction among these factors are still poorly understood.

During the healing process it is of decisive importance that the growing tissue is sufficiently provided with oxygen and nutrients. This is secured by the complicated process known as angiogenesis which leads to formation of new blood vessels in situ. This process involves the orderly migration, proliferation and differentiation of vascular cells, (Folkman, M. et al. (1987). Science 235, 442). The initiation of angiogenesis by direct stimulation of endothelial cell proliferation is the presumed responsibility of two polypeptide mitogens: The class I heparin-binding growth factor (HBGF-1), also known as acidic fibroblast growth factor, and class II heparin-binding growth factor (HBGF-II) or basic fibroblast growth factor (bFGF).

(Thomas, K.A. (1985). Proc. Natl. Acad. Sci. 82, 6409), Esch, F. (1985) ibid: 6507). These factors are not found in platelets, but basic FGF is secreted from activated monocytes/macrophages as mentioned above and have been shown to induce angiogenesis in animal models in vivo.

It is therefore clear that the initial "burst" of platelet release in connection with wounding does not involve direct angiogenic factors. However, platelet extracts are shown to be angiogenic in in vivo experiments and this can be shown to be a result of monocyte activation which in turn leads to secretion of the relevant factors mentioned above. Several attempts to isolate and characterize a non-mitogenic angiogenic factor in platelets have been done, but the nature of this factor is not disclosed in the arts (Knighton, D.R. et al., 1986, Ann. Surg. 204, 323-331).

A necessary requisite for angiogenesis to occur is the presence of heparin in the wound area, and it has been shown that removal of heparin with e.g. protamine completely abolishes angiogenesis.

Therefore any factor having the capability of recruiting monocytes from the circulation to the wounded area and subsequently activate them in addition to having heparin-binding properties, must be of extreme importance for angiogenesis and the whole repair process as well.

The present invention provides heretofore unkown proteins (the human and porcine types are hereinafter referred to as hHBP and pHBP) which are uniquely suited to stimulate angiogenesis and tissue repair for the following reasons:

a) The proteins are released from platelets when these cells are activated such as it occurs in the damaged tissue.

b) The proteins bind to heparin.

c) The proteins are chemotactic for monocytes.

d) The proteins activate monocytes morphologically towards the secretory phenotype.

e) The proteins activate monocytes in culture to excrete mitogens for fibroblasts in culture.

f) Application of the proteins gives rise to angiogenesis in the hen egg chorio-allantoic membrane model.

g) The proteins increase epithealization rate when applied to wound-chambers in experimental models in rats judged from macroscopic and histological examinations.

h) The proteins increase granulation tissue formation in the same wound-chamber model judged from macroscopic and histological examinations.

i) The proteins increase blood vessel formation in the same rat wound-chamber model judged from macroscopic examinations.

Two specific examples of hitherto unknown proteins are derived from procine and human platelets the amino acid sequence of the porcine type has been fully elucidated and covered by claim 3. The human type is strongly homologous to the porcine type as evident by comparing the amino acid sequence of the porcine type as disclosed in claim 3 with the amino acid sequence for the human type as disclosed in claim 8.

An important feature in relation to tissue repair is the strong heparin-binding properties of the proteins. Shortly after tissue damage it can be observed that connective tissue mast cells among several components of importance for the inflammation also release large amounts of heparin. (Qureshi, R. et al. (1988). The Journal of Immunology 141, 2090-2096).

The released heparin is known to bind to collagen and once this has been established, the heparin-binding proteins (HBP) described in this invention thereby become immobilized. This can form a fixed gradient and as Gustafson et al. have suggested on theoretical grounds and Carter has shown experimentally, cells tend to move up a gradient of increasing substrate adhesion. Carter has suggested that this phenomenon should be called "Haptotaxis" (Greek: haptein, to fasten; taxis, arrangement). On this basis, the cell migration involved in morphogenesis, inflammation, wound healing, tumour invasion and indeed all tissue cells movements, are considered to be the result of haptotactic responses by the cells involved (Gustafson, T. et al. (1963). Intern. Rev. Cytol, 15, 139), Carter, S.B. (1965), Nature 208, 1183-1187).

On this basis the heparin-binding proteins said herein are uniquely suited to recruit monocytes from the circulation into the damaged tissue area. The subsequent activation to secretory phenotype takes place in situ i.e. the complete cocktail of monocyte/macrophage derived cytokins releases in the neighbourhood of the damaged cells in the wound and facilitate healing.

The heparin-binding proteins herein are foreseen especially to be suitable to stimulate healing of chronic wounds in man. It is generally believed that the most important pathogenic factor for the chronic leg ulcers and decubitus in elderly patients is the lack of neovascularization (angiogenesis).

On the basis of the properties mentioned for the heparin-binding proteins external application of the proteins (preferably the human type) to chronic wounds are foreseen to accelerate healing. Ablation of macrophages slows the wound-healing response (Leibovich, S. J. et al. (1975). Am. J. Pathol. 78, 71). In the human clinic such depletion of macrophages accompanies several illnesses, and it is also often a result of therapy. Thus, severe leucocytopenia is observed in cancer patients treated with chemotherapy or radiation therapy. Slow healing after surgical treatment and occurrence of chronic ulcers is often seen in such patients. The special properties shown by the heparinbinding proteins may be of therapeutically benefits in such patient groups.

HBP may also be used in the therapy of severe burns. The lack of neovascularization results in poor healing and the damaged tissue is susceptible to infections. Therefore, a component having monocyte activating properties may be of great advantage. Activated monocytes or "macrophages" act as scavengers, and by phagocytosis they remove damaged tissue debris, a most essential function in connection with burns.

More recently, the growth regulatory role of macrophages with respect to tumor growth has received considerable attention.

High concentrations of activated macrophages are cystostatic to neoplastic cells, and this effect is directed selectively against neoplastic target cells. The putative secretory product from macrophages in this context is believed to be $TNF\alpha$ (Diegelmann, R.F. et al. 1981, Plastic and Reconstructive Surgery 1968, 107-113)

The heparin-binding proteins said herein may have therapeutic possibilities in tumor therapy. HBP injected to solid tumors can recruit circulating monocytes to the tumor area and by subsequent activation mediate cytotoxic effect.

Pharmaceutical compositions for use in the present invention in the clinic as suggested above include incorporation of human HBP into creams, ointments, gels, foams, dressing materials, patches, pads, artificial skins, aqueous vehicles for soaking gauze dressing, dry swellable powders or suture coatings.

The formulations which may be used to entrap HBP are a freeze-dried pad or a hydrocolloid occlusive dressing. It is preferred that a medical dressing which provides controlled release of HBP is used.

The gel which may be used consists of an aqueous basis which is made highly viscous by adding water-soluble etherified cellulose derivatives such as alkyl cellulose, hydroxyalkyl cellulose and alkylhydroxyalkyl celluloses, e.g. methyl cellulose, hydroxyethyl cellulose, carboxymethyl cellulose, hydroxypropylmethyl cellulose and hydroxypropyl cellulose. The hydroxyalkyl cellulose derivatives, such as hydroxypropyl cellulose, hydroxyethyl cellulose and hydroxypropylmethyl cellulose are preferred. Usually HBP is dissolved in the aqueous phase before the gelling agent is added.

A freeze-dried pad may consist of a hydroxycolloid with a coherent fibrous structure formed by lyophilization of a gel. The hydrocolloid may be a water-soluble etherified cellulose derivative as mentioned above. Usually HBP is entrapped in the pads prior to freeze-drying.

A medical dressing may be an adhesive occlusive bandage having HBP incorporated in it. The dressing comprises a sealing material, a tackifier as a continuous phase, and a discontinuous phase dispersed in the continuous phase which comprises one or more water-soluble or water-swellable compounds, such as etherified cellulose derivatives mentioned above. The ability of the discontinuous phase to swell in water gives the possibility of gradual release of previous physically entrapped HBP. HBP may also be administered in liquid formulations by subcutaneous, intermuscular or intravenious injections. Furthermore, the administration of HBP may also occur by nasal, buccal, rectal or intraperitoneal routes.

According to the invention, HBP can be produced from blood platelets, obtained from porcine or human blood. More particularly, the protein is produced by fractionation of a blood platelet extract. Column chromatography using heparin-Sepharose is expedient for this purpose. Such a chromatographic method comprising gradient elution with NaCl from 0.5 M up to 3 M of a column through which an extract of the blood platelets has been poured first, results in elution of two peaks. The first peak around 1.2 M NaCl can be measured at 280 nm as a large protein peak and is a platelet factor ($PF_4$) known per se. Around 1.8 M NaCl, the protein amount is below the detection limit in the system used, but the fractions in this area have angiogenic activity. The active fractions are purified additionally by microbore reverse phase HPLC on $C_4$ column, and at 214 nm a completely pure protein peak can be detected, and this protein is identical with the present HBP's of either porcine or human type depending on the kind of platelets used.

The HBP's may also be produced by recombinant technique. Bacteria, yeasts, fungi or mammalian cell lines may be used as hosts for the production of HBP. By transformation of the host cell with a suitable vector containing the necessary transcription- and translation signals as well as the DNA-sequence encoding HBP, production of HBP can be achieved.

A choice can be made as to whether the product should be produced intracellularly or secreted to the growth medium. Many secretion signals are known. U.S. Patent No. 4 336 336 describes for procaryotes the use of a leader sequence coding for a non-cytoplasmic protein normally transported to or beyond the cell surface resulting in transfer of the fused protein to the periplasmic space. For yeasts, Kurjan & Herskowitz, Cell (1982), 30, 933-943 describes a putative $\alpha$-factor precursor containing four tandem copies of mature $\alpha$-

factor, describing the sequence and postulating a processing mechanism. This signal sequence has been used for the secretion of a wide variety of polypeptides from the yeast Saccharomyces cerevisiae ever since the discovery of the signal sequence. Brake et al, PNAS USA, 81, (1984) 4642-4646 gives one example hereof.

Bacteria are not capable of either glycosylating proteins or, in most cases, forming the correct disulphide bridges in polypeptides of human origin.

Yeast, however, can form correct disulphide bridges, but does not glycosylate proteins in the same manner as higher eucaryotes do. Yeast mutants have been isolated, which glycosylate in a manner similar to mammalian cell lines, thus making yeast a useful host for glycosylated proteins in the future.

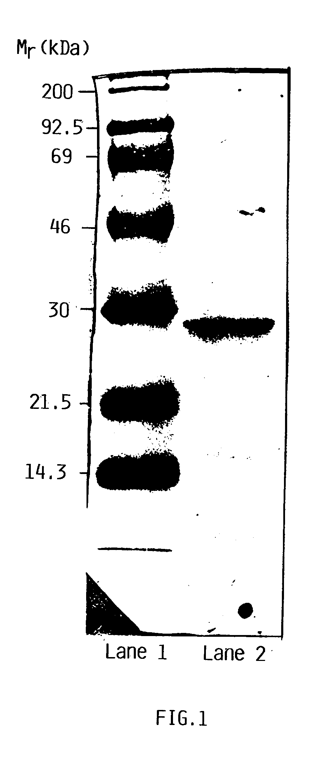

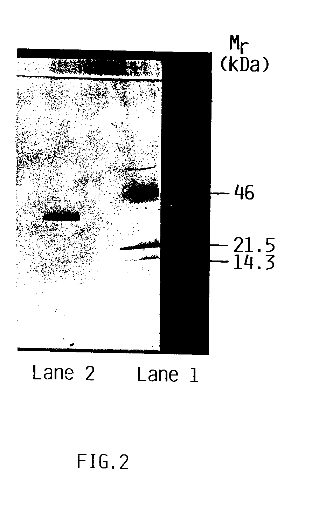

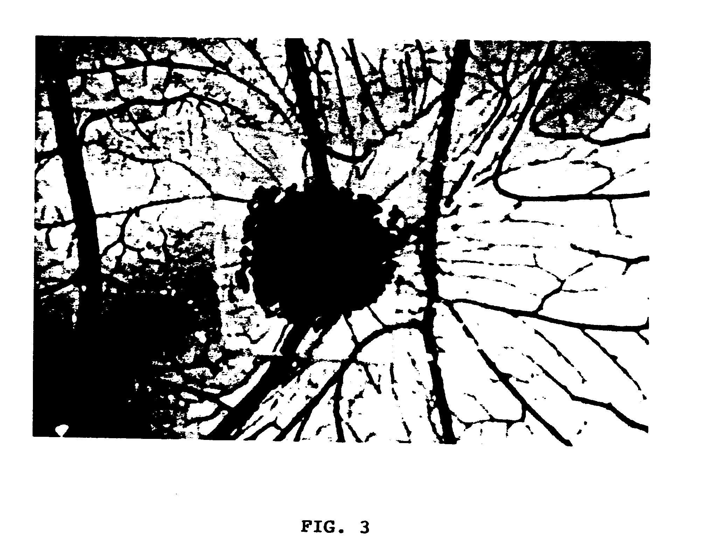

The present HBP's are moreover characterized in that they migrate as single bands in SDS-PAGE under reducing conditions, (depicted in Figs. 1 and 2) and have a $M_r$ ofabout 28 kDa.

Heparin-binding protein purified from porcine blood platelets has the following amino acid sequence:

```
 1                                              15
IleValGlyGlyArgArgAlaGlnProGlnGluPheProPheLeu


                                                30
AlaSerIleGlnLysGlnGlyArgProPheCysAlaGlyAlaLeu


                                                45
ValHisProArgPheValLeuThrAlaAlaSerCysPheArgGly


                                                60
LysAsnSerGlySerAlaSerValValLeuGlyAlaTyrAspLeu


                                                75
ArgGlnGlnGluGlnSerArgGlnThrPheSerIleArgSerIle


                                                90
SerGlnAsnGlyTyrAspProArgGlnAsnLeuAsnAspValLeu


                                               105
LeuLeuGlnLeuAspArgGluAlaArgLeuThrProSerValAla


                                               120
LeuValProLeuProProGlnAsnAlaThrValGluAlaGlyThr
```

135
AsnCysGlnValAlaGlyTrpGlyThrGlnArgLeuArgArgLeu

150
PheSerArgPheProArgValLeuArgValThrValThrSerAsn

165
ProCysLeuProArgAspMetCysIleGlyValPheSerArgArg

180
GlyArgIleSerGlnGlyAspArgGlyThrProLeuValCysAsn

195
GlyLeuAlaGlnGlyValAlaSerPheLeuArgArgArgPheXxx

196                                              210
XxxSerSerGlyPhePheThrArgValAlaLeuPheArgAsnTrp

217
IleAspSerValLeuAsnXxx

Heparin-binding protein purified from human platelets has the following sequence:
from N-terminal

1                                                15
IleValGlyGlyArgLysAlaArgProArgGlnPheProPheLeu

30
AlaSerIleGlnAsnGlnGlyArgHisPheCysGlyGlyAlaLeu

45
IleHisAlaArgPheValMetThrAlaAlaSerCysPheGlnSer

```
                                              60
GlnAsnProGlyValSerThrValValLeuGlyAlaTyrAspLeu

                                              75
ArgArgArgGluArgGlnSerArgGlnThrPheSerIleUuuUuu

                              85
MetSerGluAsnGlyTyrAspProGlnGln(.............

.....)LeuGlnLeuAspArgGluAlaXxxLeuThrSerXxxVal

ThrIleLeuProLeuPro(................)GluAlaGly

ThrArgCysGlnValAlaGlyTrpGlySerGlnArg(........

..)LeuSerArgPheProArgPheValXxxValThrValThrPro

GluAspGlnCysArgProAsnAsnValCysThrGlyValLeuThr

ArgUuuGlyGlyIleCysAsnGlyAspGlyUuuThrProValLeu

                              n-29
(..........................)SerLeuGlyProCys

GlyArgGlyProAspPhePheThrArgValAlaLeuPheArgAsp

                              n
TrpIleAspGlyValLeuAsnAsnProGly
```

The proteins are furhtermore characterized by being glycosylated.

A computer search of both heparin-binding proteins against a protein data bank using a program from the GENETIC COMPUTER GROUP, University of Wisconsin, shows that the present proteins are heretofore unknown.

The invention will be explained by the following examples:

8

Legend to Figures

| | | |
|---|---|---|
| Fig. 1 | SDS-PAGE under reducing conditions of heparinbinding protein of the porcine type. | |
| | Lane 1: Mr markers | |
| | Lane 2: pHBP | |
| Fig. 2 | SDS-PAGE under reducing conditions (see Example 2) of heparin-binding protein of the human type. | |
| | Lane 1: Mr markers | |
| | Lane 2: hHBP | |
| Fig. 3 | Test for angiogenesis for HBP by using the chick embroyo chorioallantoic membrane. For explanation, see Example 5. | |
| Fig. 4, 5 and 6 | In these pictures HBP treated monocytes (8 ng/ml) (Fig. 4), Endotoxin-treated monocytes (100 ng/ml) (Fig. 5) and PBS-treated monocytes (control cells) (Fig. 6) are shown. For explanation, see Example 6. | |

EXAMPLE 1

500 g of blood platelets from porcine blood were suspended in 1.5 l of PBS, frozen and thawed three times by means of liquid nitrogen ($N_2$), called Freeze/Thaw below. Freeze/Thaw was centrifuged at 40,000 x g for 30 minutes, and the resulting supernatant was subjected to ultracentrifugation at 300,000 x g for 60 minutes. The resulting supernatant was dialysed for 48 hours against 20 volumes of 10 mM phosphate buffer, 0.5 M NaCl, pH 7.4. The dialysate was pumped on a 5 cm (I.D.) x 10 cm Heparin-Sepharose® Cl-4B column with a flow of 120 ml/h. The column was washed with the same buffer as the sample was dialysed against (buffer A) until no more protein eluted. The column was then eluted with a linear gradient from buffer A to buffer B = 10 mM phosphate buffer, 3 M NaCl, pH 7.4 for 20 hours with a flow of 1.7 ml/min. 200 fractions (during 6 min. each) were collected, and the angiogenic effect was tested. 50 fractions distributed symmetrically around the 1.8 M NaCl fraction in the gradient showed activity. These fractions were pooled and admixed with ove-albumin until a concentration of 0.5 mg/ml. the pooled fractions were dialysed against 20 volumes of buffer A and then pumped on an 0.6 ml Heparin-Sepharose® Cl-4B column with a flow of 6 ml/h. The column was eluted with a linear gradient for 10 hours with a flow of 0.04 ml/min. 120 fractions of 200 $\mu$l each were collected and tested for angiogenic activity and then pooled. The pooled fractions were then chromatographed on a reverse phase $C_4$ column (0.1 ml volume) with a linear gradient from 0 to 80% acetonitrile containing 0.1% trifluoroacetic acid (TFA) for 30 minutes with a flow of 0.025 ml/min. The angiogenic activity was detected in a base line separated top with a retention time of 26 minutes.

SDS-PAGE under reducing conditions shows (see Fig. 1) that the peak contains one component (HBP) $M_r$ of 28 kDa. The protein in the same peak has the sequence stated in claim 4.

EXAMPLE 2

Production of HBP from human blood platelets

100 portions of fresh produced thrombocyte concentrates from healthy blood donors were mixed, and the thrombocytes centrifuged down at 1700 g for 15 min. at 25°C.

The thrombocytes centrifuged down were suspended in 3 volumes of PBS, and the suspension was frozen and thawed 6 times in liquid $N_2$. The suspension was then centrifuged at 40.000 g for 60 min. Supernatants from this were dialyzed for 2 days against 20 volumes of 10 mM phosphate buffer, 0.5 M Nacl, pH 7.4. The dialysate was pumped on a 5 cm (I.D.) x 10 cm Heparin-Sepharose® Cl-4B column with a flow of 50 ml/h. The column was washed against the same buffer as the sample was dialysed against (buffer A) until no more protein eluted. The column was the eluted with a linear gradient from buffer A to buffer B = 10 mM phosphate buffer, 3 M NaCl, pH 7.4 for 20 hours with a flow of 0.90 ml/min. 200 fractions (of 6 min. each) were collected. The fractions were examined by Microbore Reversed Phase $C_4$ column (Aquapore Butyl 100 x 2.1 mm, 7 um Brownlee Labs) with a gradient as follows:

| Time | buffer A 0.1 % TFA | buffer B 70 % $CH_3CN$ 0.085 % TFA | Flow |
|---|---|---|---|
| 0-5 min. | 60 % | 40 % | 200 ul/min. |
| 0-40 min. | 30 % | 70 % | 200 ul/min. |

The Apparatus was an Applied Biosystems 130 A Analyzer, and the protein was monitored at 214 nm. Peaks with a retention time of 20 min. were collected. After drying, the samples were diluted in 0.1 M Tris-Cl, 1 mM EDTA 2.5 % SDS, 0.01 % bromophenyl blue, 5 % 2-mercaptoethanol pH 8.0. After 5 minutes' at 95°C, the samples were subjected to SDS PAGE by means of Pharmacia Phast Gel equipment with SDS Phast Gel (8-25 %) gels and SDS buffer strips.

The samples were run at 250 V at 10 mA at 15°C in 61 Vh. (13). The fractions from 70-120 showed a band with $M_r$ 28,000. These fractions were pooled and dialysed with 20 volumes of buffer A. The dialysate was pumped on 1 ml Heparin-Sepharose® Cl-4B column and the column was eluted with a linear gradient from buffer A to buffer B for 10 hours with a flow of 0.04 ml/min. Fractions of 200 ul (120) were collected.

EXAMPLE 3

Production of HBP from human material.

For this purpose, a human cell line, K562, is used. This cell line, which originates from a chronic myeloid leucemia patient can be caused to differentiate in megakaryoblastic direction, which is a preculsor of the circulating blood platelets, with 12-0-tetradecanoyl phorbol-14-acetate (TPA). Alitalo et al. (12) has shown that the gene for PDGF is induced in these cells when they are treated with TPA.

K562 cells were cultivated in 175 $cm^3$ Nuclone bottles in RPMI 1640 medium supplemented with 10% fetal calf serum and antibiotics. The cell density was adjusted to ~ 300,000 cells per ml, and TPA (Sigma) dissolved in DMSO was added until a concentration of 3 nM. After 3 days, the cells were sedimented by centrifugation at 500 x g and then washed 1 time in 10 volumes of PBS followed by resedimentation.

10 g of cells harvested in this manner were admixed with 3 volumes of PBS, and the suspension was frozen and thawed 6 times in liquid $N_2$. The suspension was ultracentrifuged at 300,000 x g for 60 minutes, and the supernatant was then diluted at 300 ml with 10 mM Naphosphate buffer pH 7.4 containing 0.5 M NaCl. The diluted sample was pumped on an 0.5 ml Heparin-Sepharose® Cl-6B within 72 hours. The column was then eluted with a linear gradient for 10 hours from the application buffer (buffer A) to buffer B = 10 mM Naphosphate buffer pH 7.4 containing 3 M NaCl, and 120 fractions of 200 $\mu$l each were collected.

4 fractions, symmetrical around 1.8 M NaCl, were separately chromatographed on Microbore Reversed Phase $C_4$ column (Aquapore Butyl 30 x 2.1 mm, 7 $\mu$m, Brownlee Labs) with a linear gradient from 0 to 80% acetonitrile for 30 minutes admixed with 0.1% trifluoroacetic acid (TFA) and a flow of 25 $\mu$l/min.

A protein peak with a retention time of 26 minutes is identical to the retention time for the porcine HBP.

EXAMPLE 4

Detection of angiogenic properties for HBP.

Male rats of the Wistar family CRL:(WI)BR having a weight of about 240 g and being 80 days old were used. The rats were acclimatized 6 days before use at 21 ± 1°C, 60 ± 10% relative humidity with air change 10 times per hour and light from 6.30 a.m. to 6.30 p.m. The rats were kept in plastic cages with sawdust in the bottom. They were fed ad libitum with Altromin diet 1324 and had free access to drinking water.

The rats were anaesthetized with pentobarbital 50 mg/kg body weight by intraperitoneal injection. The rats were shaved on the back and disinfected. Through a 3 cm dorsal cut, the left kidney was exposed, and HBP, absorbed in advance in 10 $\mu$l of Affi/Gel® Blue 100-200 mash (wet) 75-150 $\mu$ Bio-Rad dried on a 3 x 3 mm Gelfilm® absorbable gelatine film Upjohn, was laid below the fibrous capsule of the kidney by a small incision. The surface would was closed by 5 silk sutures, and Temgesic® was given postoperatively, 0.1 ml dosed twice daily for three days. 5 days postoperatively, the rats were anaesthetized again with pentobarbital, and the left kidney was exposed. The region around the implant showed clar new vessel formation by macroscopic evaluation.

EXAMPLE 5

Test for angiogenesis for porcine and human HBP by using the chick embryo chorioallantoic membrane.

Fertilized chick eggs on the first day of gestation were placed in a humidified, 37°C incubator. At day 7 a hole was made in the blunt end of the egg using a 25GS/8 0.5 x 16 needle, and incubation continued. At day 9 a 1 cm x 1 cm "window" was cut through the shell in the pointed end of the eggs and the windows were coveted with Tegaderm® . Incubation was continued and at day 11 HBP (5-30ng), absorbed in advance in 3 $\mu$l of Affi-Gel® Blue 100-200 mesh (wet) 75-150 $\mu$ Bio-Rad dried under laminar airflow on a 3 x 3 mm sterile Gelfile® absorbable gelatine file Upjohn, was laid on the choriollantoic membrane with the affigel towards the membrane and the window was closed again using Tegaderm. After 5 days of incubation at 37°C the responses were microscopically assessed.

Both 30 and 5 ng HBP induced a strong increase in the density of the microvascular bed in the region of the affigel, with apparent regression of larger vessels and typical capillary-ball formations (see drawing).

EXAMPLE 6

Activation of human monocytes

Monocytes were isolated from "buffy coats" of citrated blood from healty blood donors.

Mononuclear cells were isolated as follows: "buffy coats" were diluted with 1 volume of cold RPMI 1640 and layered on the top of 15 ml of Ficoll-Paque in 50 ml Falcon tubes. After centrifugation at 400 x g for 30 minutes in a swingout rotor the layer between Ficoll-Paque and RPMI 1640-plasma (containing mononuclear cells and blood platelets) was removed, and the platelets were removed by washing 3 times (10 min.) at 100 x g.

The mononuclear cells were fractionated on a Percoll gradient: 10 x MEM and RPMI 1640 were added to a stock solution of Percoll (density: 1.13 g/ml) to make it isotonical with a density of 1.066 g/ml. A Percoll gradient was preformed by centrifugation at 3000 x g for 15 min. In a Hereaus medifuge with a 35° fixed angle rotor. On top of this gradient the mononuclear cells were layered and centrifugated at 2.700 x g for 20 minutes in a swingout rotor. In the upper band the monocytes were found with a purity of more than 90% as determined by non-specific esterase staining. The monocytes were cultivated at a density of 1 x $10^6$ cells/ml in 24 well macrowell dishes in RPMI 1640 containing penicillin/Streptomoycin. The medium contained less than 6 pg/ml of endotoxin.

MRC-5 human lung embryo fibroblasts were cultivated in 96 well microwell plates in MEM with 2% FCS at a starting density of 1 x $10^4$ cells/ml for 4 days before testing for mitogenic activity in monocyte culture meddium. The mitogenic activity in 100 $\mu$l of monocyte culture medium was determined by pulselabeling the MRX-5 cells with $^3$H-thymidine (1 $\mu$Ci/ml) from 24 to 42 hours after addition of monocyte medium.

Results:

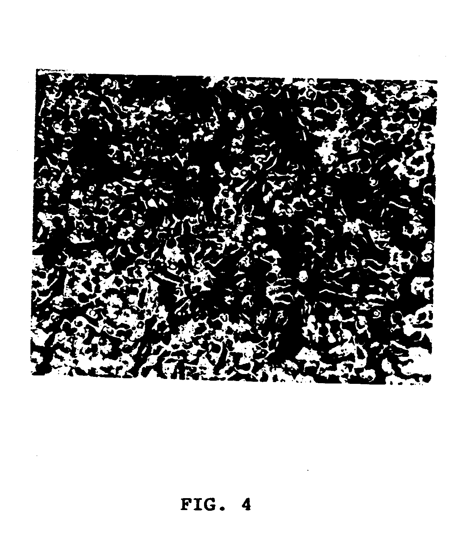

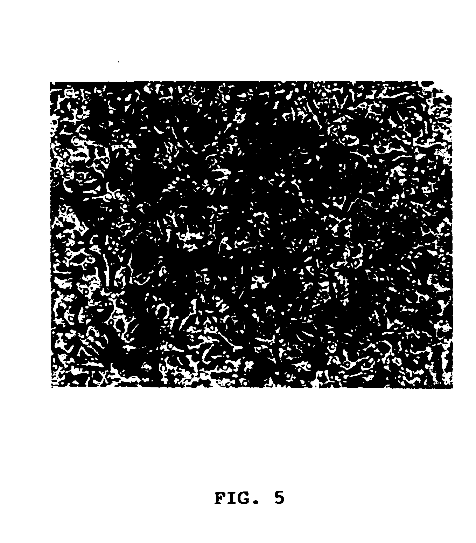

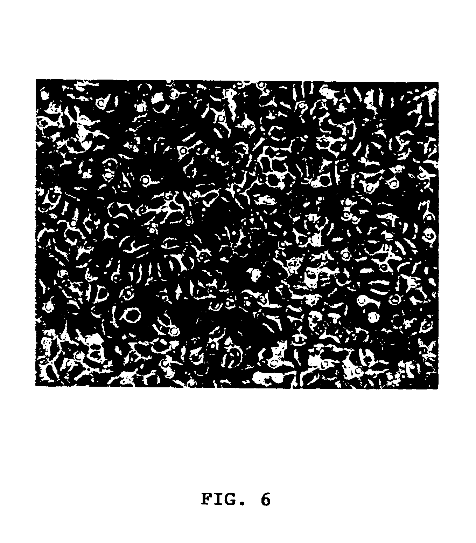

When monocytes are incubated with 5 ng HBP, a morphological change is observed after 1 and 2 days of incubation. The monocytes have become elongated (fig. 4) in a similar manner to monocytes incubated with 100 ng/ml endotoxinin containing 1 mg/ml BSA (bovine serum albumin) (fig. 5). The control cells do not have an activated morphology, as most cells still are uniformly round shaped (fig. 6). The morphological changes of the monocytes is most clearly observed, when HBP is spotted and dried on the bottom of the well before the monocytes are added. This may indicate that immobilized HBP is superior to activate monocytes compared to non-immobilized HBP.

When culture medium from monocytes is tested for mitogenic activity towards MRC-5 human fibroblasts, monocytes incubated with HBP as described above for 2 days are found to secrete about twice the amount of mitogenic activity as control monocytes do. Monocytes incubated with 100 ng/ml LPS and 21 mg/ml BSA show about 5 times the mitogenic activity as control monocytes do.

11

EXAMPLE 7

Topical HBP formulation

HBP was formulated for topical administration in the following composition:

| Ingredients | % w/w |
|---|---|
| distilled water | 92.98 |
| hydroxyethylcellulose | 4.0 |
| sodium chloride | 0.41 |
| di-sodiumhydrogenphosphate-2hydrate | 0.83 |
| potassiumdihydrogenphosphate | 0.28 |
| gelatine, hydrolyzed | 0.5 |
| benzyl alcohol | 1.0 |

to 10 g of the above mentioned composition is added 250 ng HBP.

EXAMPLE 8

Injectable compositions which are suitable for parentarel administration of HBP contain stabilizers, salts, buffers, preservatives and mixtures thereof. A simple composition which stabilizes HBP sufficiently for its biological activity to be reatined can be injected subcutaneously, intramuscularly or intraveneously.

Injectable composition

A formulation for parental administration of HBP was prepared with the following composition:

| Ingredients | % w/v |
|---|---|
| glycine | 0.15 |
| de-sodiumhydrogenephosphate | 0.026 |
| sodiumdehydrogenephosphate | 0.026 |
| mannitol | 0.74 |
| distilled water | 100 |

The formulation may also contain 0.9% (v/v) benzyl alcohol is preservative. To 1 ml of the abovementioned composition is added 25 ng of HBP.

EXAMPLE 9

Effect of porcine HBP on wound healing in rats after local administration in wound chambers.

SUMMARY

In a wound healing experiment, four groups of twentyfive rats were equipped with wound chambers after removal of the skin on the nape of the neck down to the muscular fascia, in an area of about 15 mm in diameter. The groups were dosed locally with heparin-binding protein (HBP) 12.5 ng, 2.5 ng, 0.5 ng or placebo, twice daily for eight days. A solution of 0.9% saline with 0.1% rat albumin was used as a placebo and as a dissolution medium, and all doses were administered in 100 $\mu$l volumes. At the two highest dose levels, HBP had a significant accelerating effect on the wound healing, judged on the basis of the degree of epithelialization. The wounds in the top dose group were richly vascularized.

## MATERIALS AND METHODS

### Experimental animals

100 female Wistar rats, strain CRL:(WI)BR, approximately 240 g b.wt. and 80 days old were used. The rats were purchased from Charles River, BRD, and had been acclimatized for 6 days before use at 21±1°C, 60±10% relative humidity, air change 10 times per hour and daylight from 06.30 until 18.30 h. The rats were housed singly in rectangular Orth plastic cages with pine bedding. They were fed ad libitum Altromin diet 1324 and had free access to drinking water.

### Operation

The rats were anaesthetized with pentobarbital, 50 mg/kg b.wt. intraperitoneally. They were shaved on the nape of the neck, and the operating field, 50 mm in diameter, was washed, disinfected and stripped with adhesive substance to remove small particles and hairs. Wound chambers consisting of an inner plastic ring and a nylon mesh in adhesive substance were pasted on the skin. The inner diameter of the wound chambers was 16 mm and the total diameter 45 mm. The nylon mesh was further fixated to the skin by 12 silk sutures, and the skin within the inner plastic ring was removed down to the muscular fascia. The wounds were covered with polyurethane lids pasted to the chambers with zinc plaster.

Postoperatively the rats were given Temgesic®, 0.1 ml doses twice daily for three days.

### Dosing

pHBP was administered twice daily in 100 $\mu$l volumes of 0.9% NaCl solution with 0.1% rat albumin (Sigma Λ4538). The dissolution medium was used as placebo.

Postoperatively the rats were randomized into four groups of 25 rats which were dosed as follows, once on the day of operation (day 1) and twice on day 2-8:

| Group I: | 12.5 ng pHBP |
|---|---|
| Group II: | 0.5 ng pHBP |
| Group III: | 2.5 ng pHBP |
| Group IV: | Placebo |

The doses were administered locally just underneath the wound chamber lids. The cannulae were inserted through the polyurethane lids.

### Observations

The rats were weighed on days 1, 3, 5, 7 and 9. On day 9 the wound chambers were removed during pentobarbital anaesthesia. The wounds were assessed macroscopically and photographed. The area of and around the operation site was dissected out and fixed in phosphate buffered neutral 4% formaldehyde for later histological examination. Finally, the rats were sacrificed by bleeding from the abdominal aorta. The blood was sampled for serum for IGF-I, PIIINP and hyaluronic acid analyses.

## RESULTS

Table I shows the group mean body weights for the four groups of rats. All the rats lost weight postoperatively, the lowest weights being recorded on day 5.

No significant intergroup differences in body weight were found.

As a result of the macroscopic examination of the wounds, the areas of the wounds and the areas covered with new epithelium on day 9 were calculated in the following way: The two diameters were measured, cranially-caudally and left-right, and the mean diameter ($D_{total}$) was used for calculating the total wound area:

$$\left(\frac{D_{total}}{2}\right)^2 \times 3.14 = \text{total area.}$$

The newly formed epithelium was measured from the edges, in the two places where it was widest and narrowest, respectively. The two results were added and subtracted from the $D_{total}$, giving the $D_{open}$ for the open wound.

The area for the open wound was calculated:

$$\left(\frac{D_{open}}{2}\right)^2 \times 3.14$$

and subtracted from the total area giving the area covered with new epithelium. This area was furthermore calculated as per cent of total area. In three rats dosed with 12.5 ng x 2 HBP, the unusual formation or epithelialized peninsulas in the open wound area were observed.

The results of the measurements and the calculated areas are shown in the enclosed data sheets. A survey of the results is given in Table II.

In many of the rats dosed with the highest dose of HBP (12.5 ng), a red haemorrhagic zone was observed just inside the epithelium edge and on the whole, the wounds seemed richly vascularized. A significantly higher degree of epithelialization was observed for this dose group and for the intermediate dose group (2.5 ng). The total wound area for the various groups was not significantly different from the area in the placebo group.

As the fibrin covering the wounds was removed with most of the wound chamber lids, lids + fibrin were fixed together with the tissues for histology.

CONCLUSION

On the basis of the macroscopic examination of the wounds, it can be concluded that HBP, 12.5 and 2.5 ng, administered twice daily, accelerated the wound healing significantly, as judged from the degree of epithelialization. The high degree of vascularization of the wounds in the top dose group is due to the angiogenic effect of HBP.

WOUND HEALING IN RATS

Table I

Group mean body weights in female rats with wound chambers dosed with pHBP or placebo. The rats were operated day 1.

| Preparation and dose | No. rats | Body weight, g. means ± S.E.M. | | | | |
|---|---|---|---|---|---|---|
| | | Day 1 | Day 3 | Day 5 | Day 7 | Day 9 |
| pHBP, 12.5 ng twice daily | 23 | 249 ±2 | 240 ±3 | 233 ±3 | 238 ±3 | 242 ±3 |
| pBHP, 0.5 ng twice daily | 24 | 245 ±2 | 238 ±2 | 228 ±2 | 234 ±2 | 239 ±3 |
| pHBP, 2.5 ng twice daily | 24 | 245 ±2 | 243 ±3 | 232 ±3 | 237 ±3 | 240 ±3 |
| Placebo (0.9% saline with 0.1% rat albumin | 23 | 245 ±2 | 240 ±2 | 228 ±2 | 233 ±2 | 241 ±2 |

WOUND HEALING IN RATS

Table II

| Macroscopic data obtained with pHBP | | | | | |
|---|---|---|---|---|---|
| Experiment | Placebo | Dose of pHBP administered twice daily | Total area (mm2) means ±S.E.M. | Epithelialized wound area | |
| | | | | mm2 means ±S.E.M. | % of total area means ±S.E.M. |
| VII | 0.9% saline with 0.1% rat albumin | 12.5 ng | 118.3 6.3 | 52.2* 4.1 | 46.3** 3.6 |
| | | 2.5 ng | 144.3 7.8 | 53.3* 4.2 | 39.6 3.0 |
| | | 0.5 ng | 130.2 7.4 | 44.5 3.8 | 35.8 3.1 |
| | | - (Placebo) | 125.1 6.2 | 38.5 4.2 | 31.6 3.4 |

| | | | Group I | |
|---|---|---|---|---|
| | | WOUND HEALING VII | Epithelialized Wound Area | |
| | Wound Area (total) $mm^2$ | Open Wound Area $mm^2$ | $mm^2$ | % of total area |
| 1 | 132.73 | 92.10 | 40.63 | 30.61 |
| 2 | 165.13 | 103.87 | 61.26 | 37.10 |
| 3 | 143.14 | 61.88 | 81.26 | 56.77 |
| 4 | 153.94 | 50.27 | 103.67 | 67.35 |
| 5 | 86.59 | 33.18 | 53.41 | 61.68 |
| 6 | 165.13 | 122.72 | 42.41 | 25.68 |
| 7 Dead | | | | |
| 8 | 153.94 | 86.59 | 67.35 | 43.75 |
| 9 | 95.03. | 50.27 | 44.76 | 47.10 |
| 10 | 95.03 | 44.18 | 50.85 | 53.51 |
| 11 | 113.10 | 70.88 | 42.22 | 37.33 |
| 12 | 78.54 | 50.27 | 28.27 | 35.99 |
| 13 | 143.14 | 122.72 | 20.42 | 14.27 |
| 14 | 78.54 | 28.27 | 50.27 | 64.01 |
| 15 | 132.73 | 103.87 | 28.86 | 21.74 |
| 16 | 122.72 | 95.03 | 27.69 | 22.56 |
| 17 | 122.72 | 63.62 | 59.10 | 48.16 |
| 18 | 95.03 | 28.27 | 66.76 | 70.25 |
| 19 | 103.87 | 40.18 | 63.69 | 61.32 |
| 20 | 56.75 | 12.57 | 44.18 | 77.85 |
| 21 | 143.14 | 103.87 | 39.27 | 27.44 |
| 22 | 95.03 | 50.27 | 44.76 | 47.10 |
| 23 Dead | | | | |
| 24 | 113.10 | 50.27 | 62.83 | 55.55 |
| 25 | 132.73 | 56.75 | 75.98 | 57.24 |
| X | 118.34 | | 52.17* | 46.24** |
| S.E.M. | 6.26 | | 4.05 | 3.63 |

17

| WOUND HEALING VII | | | Group II | |
| --- | --- | --- | --- | --- |
| | | | Epithelialized Wound Area | |
| | Wound Area (total) $mm^2$ | Open Wound Area $mm^2$ | $mm^2$ | % of total area |
| 26 | 201.06 | 165.13 | 35.93 | 17.87 |
| 27 | 132.73 | 86.59 | 46.14 | 34.76 |
| 28 | 113.10 | 86.59 | 26.51 | 23.44 |
| 29 | 188.69 | 113.10 | 75.59 | 40.06 |
| 30 | 132.73 | 95.03 | 37.70 | 28.40 |
| 31 | 103.87 | 70.88 | 32.99 | 31.76 |
| 32 | 143.14 | 70.88 | 72.26 | 50.48 |
| 33 | 103.87 | 44.18 | 59.69 | 57.47 |
| 34 | 122.72 | 50.27 | 72.45 | 59.04 |
| 35 | 50.27 | 28.27 | 22.00 | 43.76 |
| 36 | 132.73 | 65.75 | 75.98 | 57.24 |
| 37 | 103.87 | 78.54 | 25.33 | 24.39 |
| 38 | 95.03 | 56.75 | 38.28 | 40.28 |
| 39 | 95.03 | 63.62 | 31.41 | 33.05 |
| 40 | 86.59 | 28.27 | 58.32 | 67.35 |
| 41 | 153.94 | 113.10 | 40.84 | 26.53 |
| 42 | 113.10 | 70.88 | 42.22 | 37.33 |
| 43 | 176.71 | 153.94 | 22.77 | 12.89 |
| 44 | 103.87 | 86.59 | 17.28 | 16.64 |
| 45 | 143.14 | 78.54 | 64.60 | 45.13 |
| 46 | 143.14 | 95.03 | 48.11 | 33.61 |
| 47 | 143.14 | 103.87 | 39.27 | 27.44 |
| 48 Dead | | | | |
| 49 | 153.94 | 95.03 | 58.91 | 38.27 |
| 50 | 188.69 | 165.13 | 23.56 | 12.49 |
| $\overline{X}$ | 130.21 | | 44.51 | 35.82 |
| S.E.M. | 7.37 | | 3.79 | 3.06 |

18

| | WOUND HEALING VII | | Group III | |
|---|---|---|---|---|
| | | | Epithelialized Wound Area | |
| | Wound Area (total) $mm^2$ | Open Wound Area $mm^2$ | $mm^2$ | % of total area |
| 51 | 132.73 | 95.03 | 37.70 | 28.40 |
| 52 | 63.62 | 44.18 | 19.44 | 30.56 |
| 53 | 201.06 | 122.72 | 78.34 | 38.96 |
| 54 | 132.73 | 56.75 | 75.98 | 57.24 |
| 55 | 103.87 | 44.18 | 59.69 | 57.47 |
| 56 | 153.94 | 70.88 | 63.06 | 53.96 |
| 57 | 165.13 | 70.88 | 94.75 | 57.08 |
| 58 | 153.94 | 78.54 | 75.40 | 48.98 |
| 59 | 132.73 | 78.54 | 54.19 | 40.83 |
| 60 | 86.59 | 38.48 | 48.11 | 55.56 |
| 61 | 113.10 | 50.27 | 62.83 | 55.55 |
| 62 | 226.98 | 188.69 | 38.29 | 16.87 |
| 63 | 122.72 | 78.54 | 44.18 | 36.00 |
| 64 | 132.73 | 113.10 | 19.63 | 14.79 |
| 65 | 122.72 | 70.88 | 51.84 | 42.24 |
| 66 | 176.71 | 143.14 | 33.57 | 19.00 |
| 67 | 153.94 | 78.54 | 75.40 | 48.98 |
| 68 | 153.94 | 95.03 | 58.91 | 38.27 |
| 69 | 201.06 | 132.73 | 68.33 | 33.98 |
| 70 | 113.10 | 63.62 | 49.48 | 43.75 |
| 71 | 176.71 | 153.94 | 22.77 | 12.89 |
| 72 | 176.71 | 132.73 | 43.98 | 24.98 |
| 73 | 113.10 | 78.54 | 34.56 | 30.56 |
| 74 Dead | | | | |
| 75 | 153.94 | 103.87 | 50.07 | 32.53 |
| $\overline{X}$ | 144.33 | | 53.33* | 39.56 |
| S.E.M. | 7.75 | | 4.20 | 3.03 |

| | WOUND HEALING VII | | Group IV | |
|---|---|---|---|---|
| | Wound Area (total) $mm^2$ | Open Wound Area $mm^2$ | Epithelialized Wound Area | |
| | | | $mm^2$ | % of total area |
| 76 Dead | | | | |
| 77 | 103.87 | 70.88 | 32.99 | 31.76 |
| 78 | 95.03 | 56.75 | 38.28 | 40.28 |
| 79 | 95.03 | 38.48 | 56.55 | 59.51 |
| 80 | 70.88 | 50.27 | 70.61 | 29.08 |
| 81 | 132.73 | 56.75 | 75.98 | 57.24 |
| 82 | 86.59 | 63.62 | 22.97 | 26.53 |
| 83 | 113.10 | 95.03 | 18.07 | 15.98 |
| 84 | 176.71 | 153.94 | 22.77 | 12.89 |
| 85 | 113.10 | 78.54 | 34.56 | 30.56 |
| 86 Dead | | | | |
| 87 | 113.10 | 63.62 | 49.48 | 43.75 |
| 88 | 122.72 | 44.18 | 78.54 | 64.00 |
| 89 | 132.72 | 63.52 | 69.11 | 52.07 |
| 90 | 103.87 | 70.88 | 32.99 | 31.76 |
| 91 | 153.94 | 132.73 | 21.21 | 13.78 |
| 92 | 153.94 | 113.10 | 40.84 | 26.53 |
| 93 | 153.94 | 132.73 | 21.21 | 13.78 |
| 94 | 113.10 | 95.03 | 18.07 | 15.98 |
| 95 | 176.71 | 153.94 | 22.77 | 12.89 |
| 96 | 113.10 | 95.03 | 18.07 | 15.98 |
| 97 | 153.94 | 78.54 | 75.40 | 48.98 |
| 98 | 132.73 | 103.87 | 28.86 | 21.74 |
| 99 | 176.71 | 132.73 | 43.98 | 24.89 |
| 100 | 113.10 | 70.88 | 42.22 | 37.33 |
| $\overline{X}$ | 126.12 | | 38.50 | 31.62 |
| S.E.M. | 6.20 | | 4.19 | 3.37 |

## WOUND EXPERIMENT - MACROSCOPIC EVALUATION

| Rat No. | Wound diameter (mm) 1 | 2 | $\overline{X}$ | Epithelium edge (mm) 1 | 2 | Open wound, diameter (mm) |
|---|---|---|---|---|---|---|
| 1 | 14 | 12 | 13 | 0.5 | 0.5 | 12 (-7x3 mm) |
| 2 | 15 | 14 | 14.5 | 2 | 1 | 11.5 |
| 3 | 15 | 12 | 13.5 | 3 | 1 | 9.5 (-3x3 mm) |
| 4 | 14 | 14 | 14 | 5 | 1 | 8 |
| 5 | 11 | 10 | 10.5 | 3 | 1 | 6.5 |
| 6 | 15 | 14 | 14.5 | 2 | 0 | 12.5 |
| 7 Dead | | | | | | |
| 8 | 14 | 14 | 14 | 3 | 0.5 | 10.5 |
| 9 | 11 | 11 | 11 | 2 | 1 | 8 |
| 10 | 13 | 9 | 11 | 3 | 0.5 | 7.5 |
| 11 | 14 | 10 | 12 | 2 | 0.5 | 9.5 |
| 12 | 10 | 10 | 10 | 1.5 | 0.5 | 8 |
| 13 | 13 | 14 | 13.5 | 0.5 | 0.5 | 12.5 |
| 14 | 10 | 10 | 10 | 3 | 1 | 6 |
| 15 | 13 | 13 | 13 | 1 | 0.5 | 11.5 |
| 16 | 12 | 13 | 12.5 | 1 | 0.5 | 11 |
| 17 | 13 | 12 | 12.5 | 3 | 0.5 | 9 |
| 18 | 12 | 10 | 11 | 4 | 1 | 6 |
| 19 | 12 | 11 | 11.5 | 2 | 2 | 7.5 (-2x2 mm) |
| 20 | 8 | 9 | 8.5 | 4 | 0.5 | 4 |
| 21 | 13 | 14 | 13.5 | 1 | 1 | 11.5 |
| 22 | 11 | 11 | 11 | 2 | 1 | 8 |
| 23 Dead | | | | | | |
| 24 | 12 | 12 | 12 | 4 | 0 | 8 |
| 25 | 13 | 13 | 13 | 3 | 1.5 | 8.5 |

EP 0 409 858 B1

WOUND EXPERIMENT - MACROSCOPIC EVALUATION

| Rat No. | Wound diameter (mm) 1 | 2 | $\overline{X}$ | Epithelium edge (mm) 1 | 2 | Open wound, diameter (mm) |
|---|---|---|---|---|---|---|
| 26 | 16 | 16 | 16 | 1 | 0.5 | 14.5 |
| 27 | 12 | 14 | 13 | 1.5 | 1 | 10.5 |
| 28 | 12 | 12 | 12 | 1 | 0.5 | 10.5 |
| 29 | 16 | 15 | 15.5 | 3 | 0.5 | 12 |
| 30 | 13 | 13 | 13 | 1 | 1 | 11 |
| 31 | 12 | 12 | 11.5 | 1.5 | 0.5 | 9.5 |
| 32 | 14 | 13 | 13.5 | 2 | 2 | 9.5 |
| 33 | 11 | 12 | 11.5 | 2 | 2 | 7.5 |
| 34 | 14 | 11 | 12.5 | 4 | 0.5 | 8 |
| 35 | 8 | 8 | 8 | 1.5 | 0.5 | 6 |
| 36 | 13 | 13 | 13 | 2.5 | 2 | 8.5 |
| 37 | 13 | 10 | 11.5 | 1 | 0.5 | 10 |
| 38 | 11 | 11 | 11 | 2 | 0.5 | 8.5 |
| 39 | 10 | 12 | 11 | 1.5 | 0.5 | 9 |
| 40 | 12 | 9 | 10.5 | 2 | 2.5 | 6 |
| 41 | 14 | 14 | 14 | 1.5 | 0.5 | 12 |
| 42 | 12 | 12 | 12 | 2.5 | 0.5 | 9.5 |
| 43 | 15 | 15 | 15 | 0.5 | 0.5 | 14 |
| 44 | 11 | 12 | 11.5 | 1 | 0 | 10.5 |
| 45 | 15 | 12 | 13.5 | 2.5 | 1 | 10 |
| 46 | 14 | 13 | 13.5 | 2 | 0.5 | 11 |
| 47 | 14 | 13 | 13.5 | 2 | 0 | 11.5 |
| 48 Dead | | | | | | |
| 49 | 15 | 13 | 14 | 3 | 0 | 11 |
| 50 | 16 | 15 | 15.5 | 0.5 | 0.5 | 14.5 |

EP 0 409 858 B1

WOUND EXPERIMENT - MACROSCOPIC EVALUATION

| Rat No. | Wound diameter (mm) 1 | 2 | $\overline{X}$ | Epithelium edge (mm) 1 | 2 | Open wound, diameter (mm) |
|---|---|---|---|---|---|---|
| 51 | 14 | 12 | 13 | 1 | 1 | 11 |
| 52 | 9 | 9 | 9 | 1 | 0.5 | 7.5 |
| 53 | 16 | 16 | 16 | 3 | 1 | 8.5 |
| 54 | 14 | 12 | 13 | 3.5 | 1 | 8.5 |
| 55 | 12 | 11 | 11.5 | 2.5 | 1.5 | 7.5 |
| 56 | 14 | 14 | 14 | 3.5 | 1 | 9.5 |
| 57 | 15 | 14 | 14.5 | 4 | 1 | 9.5 |
| 58 | 15 | 13 | 14 | 3.5 | 0.5 | 10 |
| 59 | 13 | 13 | 13 | 3 | 0 | 10 |
| 60 | 11 | 10 | 10.5 | 3 | 0.5 | 7 |
| 61 | 12 | 12 | 12 | 4 | 0 | 8 |
| 62 | 17 | 17 | 17 | 1 | 0.5 | 15.5 |
| 63 | 13 | 12 | 12.5 | 2 | 0.5 | 10 |
| 64 | 12 | 14 | 13 | 1 | 0 | 12 |
| 65 | 14 | 11 | 12.5 | 3 | 0 | 9.5 |
| 66 | 15 | 15 | 15 | 1 | 0.5 | 13.5 |
| 67 | 14 | 14 | 14 | 2 | 2 | 10 |
| 68 | 13 | 15 | 14 | 2 | 1 | 11 |
| 69 | 16 | 16 | 15 | 2 | 1 | 13 |
| 70 | 12 | 12 | 12 | 2 | 1 | 9 |
| 71 | 15 | 15 | 15 | 0.5 | 0.5 | 14 |
| 72 | 16 | 14 | 15 | 1.5 | 0.5 | 13 |
| 73 | 12 | 12 | 12 | - | - | 10 |
| 74 Dead | | | | | | |
| 75 | 15 | 13 | 14 | 2 | 0.5 | 11.5 |

EP 0 409 858 B1

WOUND EXPERIMENT - MACROSCOPIC EVALUATION

| Rat No. | Wound diameter (mm) 1 | 2 | X | Epithelium edge (mm) 1 | 2 | Open wound, diameter (mm) |
|---|---|---|---|---|---|---|
| 76 Dead | | | | | | |
| 77 | 11 | 12 | 11.5 | 2 | 0 | 9.5 |
| 78 | 11 | 11 | 11 | 2 | 0.5 | 8.5 |
| 79 | 10 | 12 | 11 | 2 | 2 | 7 |
| 80 | 10 | 9 | 9.5 | 1.5 | 0 | 8 |
| 81 | 13 | 13 | 13 | 3 | 1.5 | 8.5 |
| 82 | 12 | 9 | 10.5 | 1 | 0.5 | 9 |
| 83 | 13 | 11 | 12 | 1 | 0 | 11 |
| 84 | 15 | 15 | 15 | 0.5 | 0.5 | 14 |
| 85 | 12 | 12 | 12 | 2 | 0 | 10 |
| 86 Dead | | | | | | |
| 87 | 13 | 11 | 12 | 2 | 1 | 9 |
| 88 | 12 | 13 | 12.5 | 3 | 2 | 7.5 |
| 89 | 13 | 13 | 13 | 3 | 1 | 9 |
| 90 | 12 | 11 | 11.5 | 2 | 0 | 9.5 |
| 91 | 14 | 14 | 14 | 1 | 0 | 13 |
| 92 | 14 | 14 | 14 | 2 | 0 | 12 |
| 93 | 14 | 14 | 14 | 0.5 | 0.5 | 13 |
| 94 | 14 | 10 | 12 | 1 | 0 | 11 |
| 95 | 14 | 16 | 15 | 0.5 | 0.5 | 14 |
| 96 | 13 | 13 | 13 | 1 | 1 | 11 |
| 97 | 14 | 14 | 14 | 3 | 1 | 10 |
| 98 | 13 | 13 | 13 | 1 | 0.5 | 11.5 |
| 99 | 14 | 16 | 15 | 1.5 | 0.5 | 13 |
| 100 | 11 | 13 | 12 | 2 | 0.5 | 9.5 |

Materials:

A 5 µm thick slice is cut out in the centre (craniocaudal) from the paraffin embedded wound. The slice is stained in hematoxylin-cosin and evaluated in light microscope.

Histological evaluation

EP 0 409 858 B1

Results:

As a result of the microscopic examination of the wounds, the new epithelium on day 9 was calculated in the following way:

The diameter of the total wound was measured and the diameter of the open wound was measured and subtracted:

$$\frac{\text{Total wound} - \text{Open wound}}{2} = \text{new epithelium}$$

A rating scale was used to combine a quantitative and a qualitative histological evaluation of the wound healing in rats:

$$\frac{\begin{array}{l}\text{new epithe-} \\ \text{lium} \\ \text{(mm)}\end{array} + \left(\begin{array}{l}\text{evaluation} \\ \text{of granu-} \\ \text{lation} \\ \text{tissue} \\ \text{(0--4)}\end{array} + \begin{array}{l}\text{giant} \\ \text{cells} \\ \text{(0--4)}\end{array}\right) + \begin{array}{l}\text{hight of} \\ \text{granula-} \\ \text{tion} \\ \text{tissue} \\ \text{(mm)}\end{array} + \begin{array}{l}\text{evaluation} \\ \text{of epithe-} \\ \text{lium (0--4)}\end{array}}{4} = \text{rating}$$

The areas of the wounds and the areas covered with new epithelium on day 9 were calculated in the following way:

$$\text{Total area} = \left(\frac{\text{total wound diameter}}{2}\right)^2 \times 3.14$$

New epithelium area =

$$\text{Total area} = \left(\frac{\text{open wound diameter}}{2}\right)^2 \times 3.14$$

and the new epithelium area was furthermore calculated as per cent of total area.

The results of the measurements and the calculated ratings and areas are as shown in the Tables.

CONCLUSION

On the basis of the microscopic examination of the wounds and the evaluation of the data in the rating scale, it may be concluded that p-HBP, doses 12.5 ng and 0.5 ng, has an effect on the wound healing.

The effect is seen on the granulation tissues which are high in group 1 and 2, doses 12.5 ng and 0.5 ng pHBP (table II). In group 1, dose 12.5 ng pHBP, the granulation tissues are more mature compared to the placebo group (table II). From the area calculation the degree of epithelialization shows no significant difference between the groups.

WOUND HEALING EXPERIMENT VII

## Table 1

## Microscopic data obtained with pHBP

| Group | Rating scale Mean ± S.E.M. |
|---|---|
| 1 12.5 ng p-HBP | 2.53 *** 0.06 |
| 2 0,5 ng p-HBP | 2.35 * 0.07 |
| 3 2.5 ng p-HBP | 2.26 0.07 |
| 4 Placebo | 2.10 0.09 |

*** p < 0.001 levels of significant difference from
placebo

** p < 0.01

* p < 0.05

WOUND HEALING EXPERIMENT VII

Table II

| Group | | Total wound (mm) | Open wound (mm) | Granulation tissue (0-4) | Giant cells (0-4) | Epithelium edge (mm) | Evaluation of epithelium (0-4) | Hight of granulation tissue (mm) |
|---|---|---|---|---|---|---|---|---|
| 1 12.5 ng HBP | X | 9.26* | 6.52 | 2.96* | 0.48* | 2.78 | 4.00 | 2.26** |
| | Sd | 1.91 | 2.19 | 0.21 | 1.08 | 1.13 | 0.00 | 0.54 |
| | SEM | 0.40 | 0.46 | 0.04 | 0.23 | 0.23 | 0.00 | 0.11 |
| 2 0.5 ng p-HBP | X | 9.33* | 6.88* | 2.58 | 0.58* | 2.46 | 4.00 | 2.17** |
| | Sd | 1.86 | 2.33 | 0.50 | 1.06 | 1.22 | 0.00 | 0.56 |
| | SEM | 0.38 | 0.48 | 0.10 | 0.22 | 0.25 | 0.00 | 0.12 |
| 3 2.5 ng p-HBP | X | 8.88 | 6.54 | 2.63 | 0.63 | 2.33 | 4.00 | 1.88 |
| | Sd | 2.11 | 2.06 | 0.49 | 1.28 | 1.13 | 0.00 | 0.61 |
| | SEM | 0.43 | 0.42 | 0.10 | 0.26 | 0.23 | 0.00 | 0.13 |
| 4 Placebo | X | 8.22 | 5.39 | 2.70 | 1.39 | 2.83 | 4.00 | 1.70 |
| | Sd | 1.35 | 1.85 | 0.47 | 1.50 | 1.34 | 0.00 | 0.56 |
| | SEM | 0.27 | 0.39 | 0.10 | 0.31 | 0.28 | 0.00 | 0.12 |

*** $p < 0.001$ levels of significant difference from placebo

** $p < 0.01$

* $p < 0.05$

## Claims

**Claims for the following Contracting States : BE, CH, DE, FR, GB, IT, LI, LU, NL, SE**

1. A heparin-binding protein having an apparent molecular weight in glycosylated condition of about 28 kDa, determined by SDS-PAGE under reducing conditions, the protein further exhibiting angiogenic properties in vivo and chemotactic properties against monocytes.

2. A heparin-binding protein according to claim 1 of the porcine type, **characterized** in that it is glycosylated at Asn 113.

3. A heparin-binding protein of the porcine type, **characterized** by having the amino acid sequence:

1              15

IleValGlyGlyArgArgAlaGlnProGlnGluPheProPheLeu

                30

AlaSerIleGlnLysGlnGlyArgProPheCysAlaGlyAlaLeu

                45

ValHisProArgPheValLeuThrAlaAlaSerCysPheArgGly

                60

LysAsnSerGlySerAlaSerValValLeuGlyAlaTyrAspLeu

                75

ArgGlnGlnGluGlnSerArgGlnThrPheSerIleArgSerIle

                90

SerGlnAsnGlyTyrAspProArgGlnAsnLeuAsnAspValLeu

                105

LeuLeuGlnLeuAspArgGluAlaArgLeuThrProSerValAla

                120

LeuValProLeuProProGlnAsnAlaThrValGluAlaGlyThr

                135

AsnCysGlnValAlaGlyTrpGlyThrGlnArgLeuArgArgLeu

                150

PheSerArgPheProArgValLeuArgValThrValThrSerAsn

                165

ProCysLeuProArgAspMetCysIleGlyValPheSerArgArg

                180

GlyArgIleSerGlnGlyAspArgGlyThrProLeuValCysAsn

```
                                                              195
        GlyLeuAlaGlnGlyValAlaSerPheLeuArgArgArgPheXxx


        196                                               210
        XxxSerSerGlyPhePheThrArgValAlaLeuPheArgAsnTrp


                            217
        IleAspSerValLeuAsnXxx
```

wherein X195 and X196 are arbitrary amino acids, and X217 is one or two arbitrary amino acids.

4.  A heparin-binding protein of the porcine type according to claim 3, **characterized** in that X217 is the two amino acids in the sequence AsnPro.

5.  A heparin-binding protein of the human type, **characterized** by having the following amino acid sequences:
    from N-terminal

```
        1                                               15
        IleValGlyGlyArgLysAlaArgProArgGlnPheProPheLeu


                                                        30
        AlaSerIleGlnAsnGlnGlyArgHisPheCysGlyGlyAlaLeu


                            35
        IleHisAlaArgPhe
```

    C-terminal

```
        n-15                                      n-1   n
        ValAlaLeuPheArgAspTrpIleAspGlyValLeuAsnAsnProGly
```

    wherein n denotes the total number of amino acids in the protein sequence.

6.  A heparin-binding protein according to claim 5, **characterized** by having the following amino acid sequence:
    from N-terminal

29

```
                 1                                                   15
         IleValGlyGlyArgLysAlaArgProArgGlnPheProPheLeu


                                                     30
         AlaSerIleGlnAsnGlnGlyArgHisPheCysGlyGlyAlaLeu


                                                     45
         IleHisAlaArgPheValMetThrAlaAlaSerCysPheGlnSer


                                                     60
         GlnAsnProGlyValSerThrValValLeuGlyAlaTyrAspLeu


                                             73
         ArgArgArgGluArgGlnSerArgGlnThrPheSerIle
```

C-terminal

```
           n-29                                         n-15
         SerLeuGlyProCysGlyArgGlyProAspPhePheThrArgVal


                                                         n
         AlaLeuPheArgAspTrpIleAspGlyValLeuAsnAsnProGly
```

wherein n has the significance defined in claim 5.

7. A heparin-binding protein according to claim 6 also comprising the following amino acid sequences between the amino acids Gln (69) and Ser (n-29):
from N-terminal

1                                                 15

IleValGlyGlyArgLysAlaArgProArgGlnPheProPheLeu

                                                30

AlaSerIleGlnAsnGlnGlyArgHisPheCysGlyGlyAlaLeu

                                                45

IleHisAlaArgPheValMetThrAlaAlaSerCysPheGlnSer

                                                60

GlnAsnProGlyValSerThrValValLeuGlyAlaTyrAspLeu

                                           73

ArgArgArgGluArgGlnSerArgGlnThrPheSerIle

69

GlnThrPheSerIleUuuUuuMetSerGluAsnGlyTyrAspPro

GlnGln
------

LeuGlnLeuAspArgGluAlaXxxLeuThrSerXxxValThrIle

LeuProLeuPro
------------

GluAlaGlyThrArgCysGlnValAlaGlyTrpGlySerGlnArg
------------------

LeuSerArgPheProArg
------------------

PheValXxxValThrValThrProGluAspGlnCysArgProAsn

```
AsnValCysThrGlyValLeuThrArg

--------------------------


UuuGlyGlyIleCysAsnGlyAspGlyUuuThrProValLeu

------------------------------------------
```

wherein Uuu is an unkown amino acid and Xxx is a possible glycosylation site, probably Asn.
C-terminal

```
n-29                                          n-15

SerLeuGlyProCysGlyArgGlyProAspPhePheThrArgVal


                                              n

AlaLeuPheArgAspTrpIleAspGlyValLeuAsnAsnProGly
```

wherein n has the significance defined in claim 5.

8. A heparin-binding protein according to claim 6, having the following structure:
from N-terminal

```
1                                             15

IleValGlyGlyArgLysAlaArgProArgGlnPheProPheLeu


                                              30

AlaSerIleGlnAsnGlnGlyArgHisPheCysGlyGlyAlaLeu


                                              45

IleHisAlaArgPheValMetThrAlaAlaSerCysPheGlnSer


                                              60
```

```
GlnAsnProGlyValSerThrValValLeuGlyAlaTyrAspLeu

                                             75
ArgArgArgGluArgGlnSerArgGlnThrPheSerIleUuuUuu

                        85
MetSerGluAsnGlyTyrAspProGlnGln(..............

.....)LeuGlnLeuAspArgGluAlaXxxLeuThrSerXxxVal

ThrIleLeuProLeuPro(................)GluAlaGly

ThrArgCysGlnValAlaGlyTrpGlySerGlnArg(........

..)LeuSerArgPheProArgPheValXxxValThrValThrPro

GluAspGlnCysArgProAsnAsnValCysThrGlyValLeuThr

ArgUuuGlyGlyIleCysAsnGlyAspGlyUuuThrProValLeu

                              n-29
(.........................)SerLeuGlyProCys

GlyArgGlyProAspPhePheThrArgValAlaLeuPheArgAsp

                              n
TrpIleAspGlyValLeuAsnAsnProGly
```

wherein n has the significance defined in claim 5; Uuu and Xxx have the significance defined in claim 7.

9. A therapeutic preparation, **characterized** in that it contains a therapeutically active amount of the heparin-binding protein of porcine type according to claims 1-4.

EP 0 409 858 B1

**10.** A therapeutic preparation, **characterized** in that it contains a therapeutically active amount of the heparin-binding protein of the human type according to claims 5-8.

**11.** A process for producing the heparin-binding protein of porcine type according to claims 1-4, **characterized** by extracting porcine thrombocytes, and purifying the extract by chromatography on heparin-Sepharose and Reversed Phase HPLC for isolation of heparin-binding protein.

**12.** A process for producing the heparin-binding protein of the human type according to claims 6-8, **characterized** by extracting human thrombocytes, and purifying the extract by chromatography on Heparin-Sepharose and Reversed Phase HPLC for isolation of the protein.

**13.** A process according to any of claims 11 and 12, **characterized** by performing the application of the cell extracts on heparin-Sepharose in 0.5 molar NaCl solution adjusted to a pH value of 7.2 to 7.6, preferably 7.4.

**14.** A process for producing a heparin-binding protein, according to any of claims 1-8, **characterized** by using biosynthetic recombinant technique.

**15.** A DNA structure coding for the heparin-binding protein according to any of claims 1-4, 9 and 11.

**16.** A cDNA coding for the heparin-binding protein of human type according to claims 5-8, 10 and 12.

**Claims for the following Contracting State : AT**

**1.** A process for producing a herapin-binding protein having an apparent molecular weight in glycosylated condition of about 28 kDa, determined by SDS-PAGE under reducing conditions, the protein further exhibiting angiogenic properties in vivo and chemotactic properties against monocytes, **characterized** by extracting mammal thrombocytes, and purifying the extract by chromatography on heparin-sepharose and Reverse Phase HPLC for isolation of herapin-binding protein.

**2.** A process according to claim 1, **characterized** in that thrombocytes of the porcine type are used for the extraction.

**3.** A process according to claim 1, **characterized** in that thrombocytes of the human type are used for the extraction.

**4.** A process according to claim 2 or 3, **characterized** in that the cell extracts are applied on heparin-sepharose in about 0.5 molar NaCl solution adjusted to a pH value of 7.2 to 7.6, preferably about 7.4.

**5.** A process for producing a heparin-binding protein having an apparent molecular weight in glycosylated condition of about 28 kDa, determined by SDS-PAGE under reducing conditions, the protein further exhibiting angiogenic properties in vivo and chemotactic properties against monocytes, **characterized** in that a DNA sequence coding for a heparin-binding protein as defined in claim 1 is used in a biosynthetic process for the production of the protein.

**6.** A process according to claim 5, **characterized** in that a DNA sequence is used which is coding for a protein of the porcine type having the amino acid sequence:

34

```
1                                           15
IleValGlyGlyArgArgAlaGlnProGlnGluPheProPheLeu

                                            30
AlaSerIleGlnLysGlnGlyArgProPheCysAlaGlyAlaLeu

                                            45
ValHisProArgPheValLeuThrAlaAlaSerCysPheArgGly

                                            60
LysAsnSerGlySerAlaSerValValLeuGlyAlaTyrAspLeu

                                            75
ArgGlnGlnGluGlnSerArgGlnThrPheSerIleArgSerIle

                                            90
SerGlnAsnGlyTyrAspProArgGlnAsnLeuAsnAspValLeu

                                           105
LeuLeuGlnLeuAspArgGluAlaArgLeuThrProSerValAla

                                           120
LeuValProLeuProProGlnAsnAlaThrValGluAlaGlyThr

                                           135
AsnCysGlnValAlaGlyTrpGlyThrGlnArgLeuArgArgLeu

                                           150
PheSerArgPheProArgValLeuArgValThrValThrSerAsn

                                           165
```

```
                ProCysLeuProArgAspMetCysIleGlyValPheSerArgArg


                                                        180
                GlyArgIleSerGlnGlyAspArgGlyThrProLeuValCysAsn



                                                        195
                GlyLeuAlaGlnGlyValAlaSerPheLeuArgArgArgPheXxx


                196                                      210
                XxxSerSerGlyPhePheThrArgValAlaLeuPheArgAsnTrp


                                      217
                IleAspSerValLeuAsnXxx
```

wherein X195 and X196 are arbitrary amino acids, and X217 is one or two arbitrary amino acids.

7.  A process according to claim 5, **characterized** in that a DNA sequence is used which is coding for a protein of the human type having the amino acid sequence:
    from N-terminal

```
                1                                        15
                IleValGlyGlyArgLysAlaArgProArgGlnPheProPheLeu


                                                        30
                AlaSerIleGlnAsnGlnGlyArgHisPheCysGlyGlyAlaLeu


                          35
                IleHisAlaArgPhe
```

C-terminal

```
                n-15                                   n-1   n


                ValAlaLeuPheArgAspTrpIleAspGlyValLeuAsnAsnProGly
```

wherein n denotes the total number of amino acids in the protein sequence.

8.  A process according to claim 7, **characterized** in that the DNA sequence used is coding for a protein having the following amino acid sequence:
    from N-terminal

```
1                                               15
IleValGlyGlyArgLysAlaArgProArgGlnPheProPheLeu


                                                30
AlaSerIleGlnAsnGlnGlyArgHisPheCysGlyGlyAlaLeu


                                                45
IleHisAlaArgPheValMetThrAlaAlaSerCysPheGlnSer


                                                60
GlnAsnProGlyValSerThrValValLeuGlyAlaTyrAspLeu


                                        73
ArgArgArgGluArgGlnSerArgGlnThrPheSerIle
```

C-terminal
```
n-29                                            n-15
SerLeuGlyProCysGlyArgGlyProAspPhePheThrArgVal


                                                n
AlaLeuPheArgAspTrpIleAspGlyValLeuAsnAsnProGly
```

wherein n has the significance defined in claim 5.

9. A process according to claim 7, **characterized** in that the DNA sequence used is coding for a protein having the following acid sequence:
from N-terminal

```
1                                    15
IleValGlyGlyArgLysAlaArgProArgGlnPheProPheLeu


                                     30
AlaSerIleGlnAsnGlnGlyArgHisPheCysGlyGlyAlaLeu


                                     45
IleHisAlaArgPheValMetThrAlaAlaSerCysPheGlnSer


                                     60
GlnAsnProGlyValSerThrValValLeuGlyAlaTyrAspLeu


                                73
ArgArgArgGluArgGlnSerArgGlnThrPheSerIle



69
GlnThrPheSerIleUuuUuuMetSerGluAsnGlyTyrAspPro


GlnGln
------


LeuGlnLeuAspArgGluAlaXxxLeuThrSerXxxValThrIle


LeuProLeuPro
------------


GluAlaGlyThrArgCysGlnValAlaGlyTrpGlySerGlnArg
------------------


LeuSerArgPheProArg
```

------------------

PheValXxxValThrValThrProGluAspGlnCysArgProAsn

AsnValCysThrGlyValLeuThrArg

--------------------------

UuuGlyGlyIleCysAsnGlyAspGlyUuuThrProValLeu

------------------------------------------

wherein Uuu is an unkown amino acid and Xxx is a possible glycosylation site, probably Asn.
C-terminal

n-29                                                              n-15
SerLeuGlyProCysGlyArgGlyProAspPhePheThrArgVal

                                                                    n
AlaLeuPheArgAspTrpIleAspGlyValLeuAsnAsnProGly

wherein n has the significance defined in claim 5.

**10.** A process according to claim 7, **characterized** in that the DNA sequence used is coding for a protein
having the following amino acid sequence:
from N-terminal

1                                                                15
IleValGlyGlyArgLysAlaArgProArgGlnPheProPheLeu

                                                                30
AlaSerIleGlnAsnGlnGlyArgHisPheCysGlyGlyAlaLeu

45
IleHisAlaArgPheValMetThrAlaAlaSerCysPheGlnSer

60
GlnAsnProGlyValSerThrValValLeuGlyAlaTyrAspLeu

75
ArgArgArgGluArgGlnSerArgGlnThrPheSerIleUuuUuu

85
MetSerGluAsnGlyTyrAspProGlnGln(.............

.....)LeuGlnLeuAspArgGluAlaXxxLeuThrSerXxxVal

ThrIleLeuProLeuPro(................)GluAlaGly

ThrArgCysGlnValAlaGlyTrpGlySerGlnArg(........

..)LeuSerArgPheProArgPheValXxxValThrValThrPro

GluAspGlnCysArgProAsnAsnValCysThrGlyValLeuThr

ArgUuuGlyGlyIleCysAsnGlyAspGlyUuuThrProValLeu

n-29
(..........................)SerLeuGlyProCys

GlyArgGlyProAspPhePheThrArgValAlaLeuPheArgAsp

TrpIleAspGlyValLeuAsnAsnProGly

wherein n has the significance defined in claim 5; Uuu and Xxx have the significance defined in claim 7.

**Patentansprüche**
**Patentansprüche für folgende Vertragsstaaten : BE, CH, DE, FR, GB, IT, LI, LU, NL, SE**

1. Heparin-bindendes Protein mit einem scheinbaren Molekulargewicht von ungefähr 28 kDa im glykosylierten Zustand, welches durch SDS-PAGE unter reduzierenden Bedingungen bestimmt worden ist, das Protein weiterhin angiogene Eigenschaften in vivo und chemotaktische Eigenschaften gegen Monocyten zeigt.

2. Heparin-bindendes Protein gemäß Anspruch 1 vom Porcin-Typ, dadurch gekennzeichnet, daß es an Asn 113 glykosyliert ist.

3. Heparin-bindendes Protein des Porcin-Typs, dadurch gekennzeichnet, daß es die Aminosäuresequenz:

```
1                                    15
IleValGlyGlyArgArgAlaGlnProGlnGluPheProPheLeu


                                     30
AlaSerIleGlnLysGlnGlyArgProPheCysAlaGlyAlaLeu


                                     45
ValHisProArgPheValLeuThrAlaAlaSerCysPheArgGly


                                     60
LysAsnSerGlySerAlaSerValValLeuGlyAlaTyrAspLeu
```

75
ArgGlnGlnGluGlnSerArgGlnThrPheSerIleArgSerIle

90
SerGlnAsnGlyTyrAspProArgGlnAsnLeuAsnAspValLeu

105
LeuLeuGlnLeuAspArgGluAlaArgLeuThrProSerValAla

120
LeuValProLeuProProGlnAsnAláThrValGluAlaGlyThr

135
AsnCysGlnValAlaGlyTrpGlyThrGlnArgLeuArgArgLeu

150
PheSerArgPheProArgValLeuArgValThrValThrSerAsn

165
ProCysLeuProArgAspMetCysIleGlyValPheSerArgArg

180
GlyArgIleSerGlnGlyAspArgGlyThrProLeuValCysAsn

195
GlyLeuAlaGlnGlyValAlaSerPheLeuArgArgArgPheXxx

196                                    210
XxxSerSerGlyPhePheThrArgValAlaLeuPheArgAsnTrp

217
IleAspSerValLeuAsnXxx

worin X195 und X196 beliebige Aminosäuren sind und X217 eine oder zwei beliebige Aminosäuren ist, aufweist.

4. Heparin-bindendes Protein des Porcin-Typs gemäß Anspruch 3, dadurch gekennzeichnet, daß X217 zwei Aminosäuren mit der Sequenz AsnPro ist.

5. Heparin-bindendes Protein des Human-Typs, dadurch gekennzeichnet, daß es die folgenden Aminosäurensequenzen:

42

vom N-terminalen Ende

  1                                        15

IleValGlyGlyArgLysAlaArgProArgGlnPheProPheLeu

                                        30

AlaSerIleGlnAsnGlnGlyArgHisPheCysGlyGlyAlaLeu

                  35

IleHisAlaArgPhe

C-terminalen Ende

  n-15                                n-1   n

ValAlaLeuPheArgAspTrpIleAspGlyValLeuAsnAsnProGly

worin n die Gesamtzahl der Aminosäuren in der Proteinsequenz bezeichnet, aufweist.

**6.** Heparin-bindendes Protein gemäß Anspruch 5, dadurch gekennzeichnet, daß es die folgende Aminosäurensequenz:
vom N-terminalen Ende

  1                                        15

IleValGlyGlyArgLysAlaArgProArgGlnPheProPheLeu

                                        30

AlaSerIleGlnAsnGlnGlyArgHisPheCysGlyGlyAlaLeu

                                        45

IleHisAlaArgPheValMetThrAlaAlaSerCysPheGlnSer

                                      60

GlnAsnProGlyValSerThrValValLeuGlyAlaTyrAspLeu

                                      73

ArgArgArgGluArgGlnSerArgGlnThrPheSerIle

C-terminalen Ende

n-29                                                          n-15

SerLeuGlyProCysGlyArgGlyProAspPhePheThrArgVal

n

AlaLeuPheArgAspTrpIleAspGlyValLeuAsnAsnProGly

worin n die in Anspruch 5 definierte Bedeutung hat, aufweist.

7.  Heparin-bindendes Protein gemäß Anspruch 6, das ebenfalls die folgenden Aminosäurensequenzen zwischen den Aminosäuren Gln (69) und Ser (n-29):
vom N-terminalen Ende

1                                                            15

IleValGlyGlyArgLysAlaArgProArgGlnPheProPheLeu

30

AlaSerIleGlnAsnGlnGlyArgHisPheCysGlyGlyAlaLeu

45

IleHisAlaArgPheValMetThrAlaAlaSerCysPheGlnSer

60

GlnAsnProGlyValSerThrValValLeuGlyAlaTyrAspLeu

73

ArgArgArgGluArgGlnSerArgGlnThrPheSerIle

69

GlnThrPheSerIleUuuUuuMetSerGluAsnGlyTyrAspPro

GlnGln

------

LeuGlnLeuAspArgGluAlaXxxLeuThrSerXxxValThrIle

LeuProLeuPro

-----------

GluAlaGlyThrArgCysGlnValAlaGlyTrpGlySerGlnArg

------------------

LeuSerArgPheProArg

------------------

PheValXxxValThrValThrProGluAspGlnCysArgProAsn

AsnValCysThrGlyValLeuThrArg

-------------------------

UuuGlyGlyIleCysAsnGlyAspGlyUuuThrProValLeu

-------------------------------------------

worin Uuu eine unbekannte Aminosäure ist und Xxx eine mögliche Glykosylierungsstelle, vermutlich Asn, ist;

C-terminalen Ende

n-29                                                        n-15

SerLeuGlyProCysGlyArgGlyProAspPhePheThrArgVal

n

AlaLeuPheArgAspTrpIleAspGlyValLeuAsnAsnProGly

worin n die in Anspruch 5 definierte Bedeutung hat, aufweist.

8. Heparin-bindendes Protein gemäß Anspruch 6 mit der folgenden Struktur:
vom N-terminalen Ende

1                                                          15
IleValGlyGlyArgLysAlaArgProArgGlnPheProPheLeu

                                                           30
AlaSerIleGlnAsnGlnGlyArgHisPheCysGlyGlyAlaLeu

                                                           45
IleHisAlaArgPheValMetThrAlaAlaSerCysPheGlnSer

                                                           60

GlnAsnProGlyValSerThrValValLeuGlyAlaTyrAspLeu

                                                           75
ArgArgArgGluArgGlnSerArgGlnThrPheSerIleUuuUuu

                                 85
MetSerGluAsnGlyTyrAspProGlnGln(. . . . . . . . . . . . . .

. . . . . )LeuGlnLeuAspArgGluAlaXxxLeuThrSerXxxVal

ThrIleLeuProLeuPro(. . . . . . . . . . . . . . . . )GluAlaGly

ThrArgCysGlnValAlaGlyTrpGlySerGlnArg(. . . . . . . .

. . )LeuSerArgPheProArgPheValXxxValThrValThrPro

GluAspGlnCysArgProAsnAsnValCysThrGlyValLeuThr

```
ArgUuuGlyGlyIleCysAsnGlyAspGlyUuuThrProValLeu


                                 n-29
            (.............................)SerLeuGlyProCys



            GlyArgGlyProAspPhePheThrArgValAlaLeuPheArgAsp



                                    n
            TrpIleAspGlyValLeuAsnAsnProGly
```

worin n die in Anspruch 5 definierte Bedeutung hat; Uuu und Xxx die in Anspruch 7 definierte Bedeutung haben.

9. Therapeutisches Präparat, dadurch gekennzeichnet, daß es eine therapeutisch aktive Menge des Heparin-bindenden Proteins des Porcin-Typs gemäß den Ansprüchen 1-4 enthält.

10. Therapeutisches Präparat, dadurch gekennzeichnet, daß es eine therapeutisch aktive Menge des Heparin-bindenden Proteins des Human-Typs gemäß den Ansprüchen 5-8 enthält.

11. Verfahren zum Herstellen des Heparin-bindenden Proteins des Porcin-Typs gemäß den Ansprüchen 1-4, dadurch gekennzeichnet, daß zur Isolierung des Heparin-bindenden Proteins Porcin-Thrombozyten extrahiert werden und der Extrakt durch Chromatographie an Heparin-Sepharose und Reversed-Phase-HPLC gereinigt wird.

12. Verfahren zum Herstellen des Heparin-bindenden Proteins des Human-Typs gemäß den Ansprüchen 6-8, dadurch gekennzeichnet, daß zur Isolierung des Proteins Human-Thrombozyten extrahiert werden und der Extrakt durch Chromatographie an Heparin-Sepharose und Reversed-Phase-HPLC gereinigt wird.

13. Verfahren gemäß einem der Ansprüche 11 und 12, dadurch gekennzeichnet, daß das Aufbringen der Zellextrakte auf Heparin-Sepharose in 0,5-molarer NaCl-Lösung, die auf einen pH-Wert von 7,2 bis 7,6, vorzugsweise 7,4, eingestellt ist, durchgeführt wird.

14. Verfahren zum Herstellen eines Heparin-bindenden Proteins gemäß irgendeinem der Ansprüche 1-8, dadurch gekennzeichnet, daß biosynthetische rekombinante Techniken verwendet werden.

15. DNA-Struktur, die das Heparin-bindende Protein gemäß irgendeinem der Ansprüche 1-4, 9 und 11 codiert.

16. cDNA, die das Heparin-bindende Protein des Human-Typs gemäß den Ansprüchen 5-8, 10 und 12 codiert.

**Patentansprüche für folgenden Vertragsstaat : AT**

1. Verfahren zum Herstellen eines Heparin-bindenden Proteins mit einem scheinbaren Molekulargewicht von ungefähr 28 kDa im glykosylierten Zustand, welches durch SDS-PAGE unter reduzierenden Bedingungen bestimmt worden ist, das Protein darüber hinaus angiogene Eigenschaften in vivo und chemotaktische Eigenschaften gegen Monocyten aufweist, dadurch gekennzeichnet, daß zur Isolierung des Heparin-bindenden Proteins Säuger-Thrombozyten extrahiert werden und der Extrakt durch Chromatographie an Heparin-Sepharose und Reverse-Phase-HPLC gereinigt wird.

2. Verfahren gemäß Anspruch 1, dadurch gekennzeichnet, daß Thrombozyten des Porcin-Typs für die Extraktion verwendet werden.

3. Verfahren gemäß Anspruch 1, dadurch gekennzeichnet, daß Thrombozyten des Human-Typs für die Extraktion verwendet werden.

4. Verfahren gemäß Anspruch 2 oder 3, dadurch gekennzeichnet, daß die Zellextrakte auf Heparin-Sepharose in ungefähr 0,5-molarer NaCl-Lösung, die auf einen pH-Wert von 7,2 bis 7,6, vorzugsweise ungefähr 7,4, eingestellt ist, aufgebracht werden.

5. Verfahren zur Herstellung eines Heparin-bindenden Proteins mit einem scheinbaren Molekulargewicht von ungefähr 28 kDa im glykosylierten Zustand, der durch SDS-PAGE unter reduzierenden Bedingungen bestimmt worden ist, das Protein darüber hinaus angiogene Eigenschaften in vivo und chemotaktische Eigenschaften gegen Monocyten aufweist, dadurch gekennzeichnet, daß eine DNA-Sequenz, die ein Heparinbindendes Protein wie in Anspruch 1 definiert codiert, in einem biosynthetischen Verfahren zur Herstellung des Proteins verwendet wird.

6. Verfahren gemäß Anspruch 5, dadurch gekennzeichnet, daß eine DNA-Sequenz verwendet wird, die ein Protein des Porcin-Typs mit der Aminosäurensequenz:

```
1                                                    15
IleValGlyGlyArgArgAlaGlnProGlnGluPheProPheLeu


                                                     30
AlaSerIleGlnLysGlnGlyArgProPheCysAlaGlyAlaLeu


                                                     45
ValHisProArgPheValLeuThrAlaAlaSerCysPheArgGly


                                                     60
LysAsnSerGlySerAlaSerValValLeuGlyAlaTyrAspLeu


                                                     75
ArgGlnGlnGluGlnSerArgGlnThrPheSerIleArgSerIle


                                                     90
SerGlnAsnGlyTyrAspProArgGlnAsnLeuAsnAspValLeu


                                                    105
LeuLeuGlnLeuAspArgGluAlaArgLeuThrProSerValAla
```

                                                                120
LeuValProLeuProProGlnAsnAlaThrValGluAlaGlyThr

                                                                135
AsnCysGlnValAlaGlyTrpGlyThrGlnArgLeuArgArgLeu

                                                                150
PheSerArgPheProArgValLeuArgValThrValThrSerAsn

                                                                165

ProCysLeuProArgAspMetCysIleGlyValPheSerArgArg

                                                                180
GlyArgIleSerGlnGlyAspArgGlyThrProLeuValCysAsn

                                                                195
GlyLeuAlaGlnGlyValAlaSerPheLeuArgArgArgPheXxx

196                                                             210
XxxSerSerGlyPhePheThrArgValAlaLeuPheArgAsnTrp

                        217
IleAspSerValLeuAsnXxx

worin X195 und X196 beliebige Aminosäuren sind und X217 eine oder zwei beliebige Aminosäuren ist, codiert.

**7.** Verfahren gemäß Anspruch 5, dadurch gekennzeichnet, daß eine DNA-Sequenz verwendet wird, die ein Protein des Human-Typs mit der Aminosäurensequenz:
vom N-terminalen Ende

1                  15

IleValGlyGlyArgLysAlaArgProArgGlnPheProPheLeu

                  30

AlaSerIleGlnAsnGlnGlyArgHisPheCysGlyGlyAlaLeu

      35

IleHisAlaArgPhe

C-terminalen Ende

n-15                         n-1  n

ValAlaLeuPheArgAspTrpIleAspGlyValLeuAsnAsnProGly

worin n die Gesamtzahl der Aminosäuren in der Proteinsequenz bezeichnet, codiert.

8. Verfahren gemäß Anspruch 7, dadurch gekennzeichnet, daß die verwendete DNA-Sequenz ein Protein mit der folgenden Aminosäurensequenz:

vom N-terminalen Ende

1                  15

IleValGlyGlyArgLysAlaArgProArgGlnPheProPheLeu

                  30

AlaSerIleGlnAsnGlnGlyArgHisPheCysGlyGlyAlaLeu

                  45

IleHisAlaArgPheValMetThrAlaAlaSerCysPheGlnSer

                  60

GlnAsnProGlyValSerThrValValLeuGlyAlaTyrAspLeu

                  73

ArgArgArgGluArgGlnSerArgGlnThrPheSerIle

C-terminalen Ende

n-29                        n-15

SerLeuGlyProCysGlyArgGlyProAspPhePheThrArgVal

                  n

AlaLeuPheArgAspTrpIleAspGlyValLeuAsnAsnProGly

worin n die in Anspruch 5 definierte Bedeutung hat, codiert.

9. Verfahren gemäß Anspruch 7, dadurch gekennzeichnet, daß die verwendete DNA-Sequenz ein Protein mit der folgenden Aminosäurensequenz:
vom N-terminalen Ende

```
    1                                           15
    IleValGlyGlyArgLysAlaArgProArgGlnPheProPheLeu


                                                30
    AlaSerIleGlnAsnGlnGlyArgHisPheCysGlyGlyAlaLeu


                                                45
    IleHisAlaArgPheValMetThrAlaAlaSerCysPheGlnSer


                                                60
    GlnAsnProGlyValSerThrValValLeuGlyAlaTyrAspLeu


                                    73
    ArgArgArgGluArgGlnSerArgGlnThrPheSerIle


    69
    GlnThrPheSerIleUuuUuuMetSerGluAsnGlyTyrAspPro


    GlnGln
    ------


    LeuGlnLeuAspArgGluAlaXxxLeuThrSerXxxValThrIle


    LeuProLeuPro
    ------------
```

GluAlaGlyThrArgCysGlnValAlaGlyTrpGlySerGlnArg

- - - - - - - - - - - - - - - - - -

LeuSerArgPheProArg

- - - - - - - - - - - - - - - - - -

PheValXxxValThrValThrProGluAspGlnCysArgProAsn

AsnValCysThrGlyValLeuThrArg

- - - - - - - - - - - - - - - - - - - - - - - - - -

UuuGlyGlyIleCysAsnGlyAspGlyUuuThrProValLeu

- - - - - - - - - - - - - - - - - - - - - - - - - - - - - - - - - - - - - -

worin Uuu eine unbekannte Aminosäure ist und Xxx eine mögliche Glykosylierungsstelle, möglicherweise Asn, ist;

C-terminalen Ende

n−29                                                                      n−15

SerLeuGlyProCysGlyArgGlyProAspPhePheThrArgVal

n

AlaLeuPheArgAspTrpIleAspGlyValLeuAsnAsnProGly

worin n die in Anspruch 5 definierte Bedeutung hat, codiert.

**10.** Verfahren gemäß Anspruch 7, dadurch gekennzeichnet, daß die verwendete DNA-Sequenz ein Protein mit der folgenden Aminosäurensequenz:

vom N-terminalen Ende

1                                                                      15

IleValGlyGlyArgLysAlaArgProArgGlnPheProPheLeu

30

AlaSerIleGlnAsnGlnGlyArgHisPheCysGlyGlyAlaLeu

45
IleHisAlaArgPheValMetThrAlaAlaSerCysPheGlnSer

60
GlnAsnProGlyValSerThrValValLeuGlyAlaTyrAspLeu

75
ArgArgArgGluArgGlnSerArgGlnThrPheSerIleUuuUuu

85
MetSerGluAsnGlyTyrAspProGlnGln(..............

.....)LeuGlnLeuAspArgGluAlaXxxLeuThrSerXxxVal

ThrIleLeuProLeuPro(................)GluAlaGly

ThrArgCysGlnValAlaGlyTrpGlySerGlnArg(........

..)LeuSerArgPheProArgPheValXxxValThrValThrPro

GluAspGlnCysArgProAsnAsnValCysThrGlyValLeuThr

ArgUuuGlyGlyIleCysAsnGlyAspGlyUuuThrProValLeu

n-29
(............................)SerLeuGlyProCys

GlyArgGlyProAspPhePheThrArgValAlaLeuPheArgAsp

n
TrpIleAspGlyValLeuAsnAsnProGly

worin n die in Anspruch 5 definierte Bedeutung hat; Uuu und Xxx die in Anspruch 7 definierte Bedeutung haben, codiert.

**Revendications**
**Revendications pour les Etats contractants suivants : BE, CH, DE, FR, GB, IT, LI, LU, NL, SE**

1. Protéine fixant l'héparine, ayant un poids moléculaire apparent, à l'état glycosylé, d'environ 28 kDa, déterminé par électrophorèse sur gel de polyacrylamide au dodécylsulfate de sodium dans des conditions réductrices, la protéine présentant en outre des propriétés angiogènes in vivo et des propriétés chimiotactiques contre les monocytes.

2. Protéine fixant l'héparine suivant la revendication 1, de type porcin, caractérisée en ce qu'elle est glycosylée au niveau de Asn 113.

3. Protéine fixant l'héparine, de type porcin, caractérisée en ce qu'elle possède la séquence d'amino-acides :

```
1                                              15
IleValGlyGlyArgArgAlaGlnProGlnGluPheProPheLeu

                                               30
AlaSerIleGlnLysGlnGlyArgProPheCysAlaGlyAlaLeu

                                               45
ValHisProArgPheValLeuThrAlaAlaSerCysPheArgGly

                                               60
LysAsnSerGlySerAlaSerValValLeuGlyAlaTyrAspLeu

                                               75
ArgGlnGlnGluGlnSerArgGlnThrPheSerIleArgSerIle

                                               90
SerGlnAsnGlyTyrAspProArgGlnAsnLeuAsnAspValLeu

                                              105
LeuLeuGlnLeuAspArgGluAlaArgLeuThrProSerValAla

                                              120
LeuValProLeuProProGlnAsnAlaThrValGluAlaGlyThr

                                              135
AsnCysGlnValAlaGlyTrpGlyThrGlnArgLeuArgArgLeu

                                              150
PheSerArgPheProArgValLeuArgValThrValThrSerAsn

                                              165
ProCysLeuProArgAspMetCysIleGlyValPheSerArgArg

                                              180
GlyArgIleSerGlnGlyAspArgGlyThrProLeuValCysAsn
```

$$195$$

GlyLeuAlaGlnGlyValAlaSerPheLeuArgArgArgPheXxx

$$196 \qquad\qquad 210$$

XxxSerSerGlyPhePheThrArgValAlaLeuPheArgAsnTrp

$$217$$

IleAspSerValLeuAsnXxx

dans laquelle X195 et X196 sont des amino-acides arbitraires, et X217 représente un ou deux amino-acides arbitraires.

4. Protéine fixant l'héparine, de type porcin, suivant la revendication 3, caractérisée en ce que X217 représente les deux amino-acides dans la séquence AsnPro.

5. Protéine fixant l'héparine, de type humain, caractérisée en ce qu'elle possède les séquences d'amino-acides suivantes :
à partir de l'extrémité N-terminale

$$1 \qquad\qquad 15$$

IleValGlyGlyArgLysAlaArgProArgGlnPheProPheLeu

$$30$$

AlaSerIleGlnAsnGlnGlyArgHisPheCysGlyGlyAlaLeu

$$35$$

IleHisAlaArgPhe

à partir de l'extrémité C-terminale

$$n-15 \qquad\qquad\qquad n-1 \quad n$$

ValAlaLeuPheArgAspTrpIleAspGlyValLeuAsnAsnProGly

$n$ désignant le nombre total d'amino-acides dans la séquence de la protéine.

6. Protéine fixant l'héparine suivant la revendication 5, caractérisée en ce qu'elle possède la séquence d'amino-acides suivante :
à partir de l'extrémité N-terminale

1                                                                                    15
IleValGlyGlyArgLysAlaArgProArgGlnPheProPheLeu

                                                                                     30
AlaSerIleGlnAsnGlnGlyArgHisPheCysGlyGlyAlaLeu

                                                                                     45
IleHisAlaArgPheValMetThrAlaAlaSerCysPheGlnSer

                                                                                     60
GlnAsnProGlyValSerThrValValLeuGlyAlaTyrAspLeu

                                                                                     73
ArgArgArgGluArgGlnSerArgGlnThrPheSerIle

à partir de l'extrémité C-terminale
n-29                                                                               n-15
SerLeuGlyProCysGlyArgGlyProAspPhePheThrArgVal

                                                                                     n
AlaLeuPheArgAspTrpIleAspGlyValLeuAsnAsnProGly

n répondant à la définition suivant la revendication 5.

7. Protéine fixant l'héparine suivant la revendication 6, comprenant également les séquences d'amino-acides suivantes entre les amino-acides Gln (69) et Ser (n-29) :
   à partir de l'extrémité N-terminale

1                                                          15
IleValGlyGlyArgLysAlaArgProArgGlnPheProPheLeu

                                                           30
AlaSerIleGlnAsnGlnGlyArgHisPheCysGlyGlyAlaLeu

                                                           45
IleHisAlaArgPheValMetThrAlaAlaSerCysPheGlnSer

                                                           60
GlnAsnProGlyValSerThrValValLeuGlyAlaTyrAspLeu

                                                  73
ArgArgArgGluArgGlnSerArgGlnThrPheSerIle


69
GlnThrPheSerIleUuuUuuMetSerGluAsnGlyTyrAspPro

GlnGln
------

LeuGlnLeuAspArgGluAlaXxxLeuThrSerXxxValThrIle

LeuProLeuPro
-----------

GluAlaGlyThrArgCysGlnValAlaGlyTrpGlySerGlnArg
------------------

LeuSerArgPheProArg
------------------

PheValXxxValThrValThrProGluAspGlnCysArgProAsn

```
AsnValCysThrGlyValLeuThrArg
---------------------------
```

```
UuuGlyGlyIleCysAsnGlyAspGlyUuuThrProValLeu
------------------------------------------
```

séquence dans laquelle Uuu représente un amino-acide inconnu et Xxx représente un site de glycosylation possible, probablement Asn ;
à partir de l'extrémité C-terminale

```
n-29                                        n-15
SerLeuGlyProCysGlyArgGlyProAspPhePheThrArgVal
```

```
                                              n
AlaLeuPheArgAspTrpIleAspGlyValLeuAsnAsnProGly
```

séquence dans laquelle n répond à la définition suivant la revendication 5.

8.   Protéine fixant l'héparine suivant la revendication 6, ayant la structure suivante :
à partir de l'extrémité N-terminale

```
1                                            15
IleValGlyGlyArgLysAlaArgProArgGlnPheProPheLeu
```

```
                                             30
AlaSerIleGlnAsnGlnGlyArgHisPheCysGlyGlyAlaLeu
```

```
                                             45
IleHisAlaArgPheValMetThrAlaAlaSerCysPheGlnSer
```

```
                                             60
```

```
GlnAsnProGlyValSerThrValValLeuGlyAlaTyrAspLeu

                                              75
ArgArgArgGluArgGlnSerArgGlnThrPheSerIleUuuUuu


                      85
MetSerGluAsnGlyTyrAspProGlnGln(.............


.....)LeuGlnLeuAspArgGluAlaXxxLeuThrSerXxxVal


ThrIleLeuProLeuPro(................)GluAlaGly


ThrArgCysGlnValAlaGlyTrpGlySerGlnArg(........


..)LeuSerArgPheProArgPheValXxxValThrValThrPro


GluAspGlnCysArgProAsnAsnValCysThrGlyValLeuThr


ArgUuuGlyGlyIleCysAsnGlyAspGlyUuuThrProValLeu


                      n-29
(.........................)SerLeuGlyProCys


GlyArgGlyProAspPhePheThrArgValAlaLeuPheArgAsp


                      n
TrpIleAspGlyValLeuAsnAsnProGly
```

dans laquelle n répond à la définition suivant la revendication 5 ; Uuu et Xxx répondent aux définitions suivant la revendication 7.

9. Préparation thérapeutique, caractérisée en ce qu'elle contient une quantité thérapeutiquement active de la protéine fixant l'héparine, de type porcin, suivant les revendications 1 à 4.

**10.** Préparation thérapeutique, caractérisée en ce qu'elle contient une quantité thérapeutiquement active de la protéine fixant l'héparine, de type humain, suivant les revendications 5 à 8.

**11.** Procédé de production de la protéine fixant l'héparine, de type porcin, suivant les revendications 1 à 4, caractérisé par l'extraction de thrombocytes de porc, et purification de l'extrait par chromatographie sur héparine-Sepharose et par CLHP à inversion de phase pour isoler la protéine fixant l'héparine.

**12.** Procédé de production de la protéine fixant l'héparine, de type humain, suivant les revendications 6 à 8, caractérisé par l'extraction de thrombocytes humains, et la purification de l'extrait par chromatographie sur héparine-Sepharose et par CLHP à inversion de phase pour isoler la protéine.

**13.** Procédé suivant l'une quelconque des revendications 11 et 12, caractérisé en ce que l'application des extraits cellulaires sur héparine-Sepharose est effectuée dans une solution de NaCl 0,5 molaire ajustée à une valeur de pH de 7,2 à 7,6, de préférence de 7,4.

**14.** Procédé de production d'une protéine fixant l'héparine suivant l'une quelconque des revendications 1 à 8, caractérisé par l'utilisation d'une technique de formation d'un recombinant biosynthétique.

**15.** Structure d'ADN codant pour la protéine fixant l'héparine suivant l'une quelconque des revendications 1 à 4, 9 et 11.

**16.** ADNc codant pour la protéine fixant l'héparine, de type humain, suivant les revendications 5 à 8, 10 et 12.

**Revendications pour l'Etat contractant suivant : AT**

**1.** Procédé de production d'une protéine fixant l'héparine, ayant un poids moléculaire apparent, à l'état glycosylé, d'environ 28 kDa, déterminé par l'électrophorèse sur gel de polyacrylamide au dodécylsulfate de sodium dans des conditions réductrices, la protéine présentant en outre des propriétés angiogènes in vivo et des propriétés chimiotactiques contre les monocytes, caractérisé par l'extraction de thrombocytes de mammifères, et la purification de l'extrait par chromatographie sur héparine-Sepharose et CLHP à inversion de phase pour isoler la protéine fixant l'héparine.

**2.** Procédé suivant la revendication 1, caractérisé en ce que des thrombocytes de type porcin sont utilisés pour l'extraction.

**3.** Procédé suivant la revendication 1, caractérisé en ce que des thrombocytes de type humain sont utilisés par l'extraction.

**4.** Procédé suivant la revendication 2 ou 3, caractérisé en ce que les extraits cellulaires sont appliqués sur héparine-Sepharose dans une solution de NaCl approximativement 0,5 molaire ajustée à une valeur de pH de 7,2 à 7,6, de préférence d'environ 7,4.

**5.** Procédé de production d'une protéine fixant l'héparine ayant un poids moléculaire apparent, à l'état glycosylé, d'environ 28 kDa, déterminé par EGPA-SDS dans les conditions réductrices, la protéine présentant en outre des propriétés angiogènes in vivo et des propriétés chimiotactiques contre les monocytes, caractérisé en ce qu'une séquence d'ADN codant pour une protéine fixant l'héparine répondant à la définition suivant la revendication 1 est utilisée dans un procédé de biosynthèse pour la production de la protéine.

**6.** Procédé suivant la revendication 5, caractérisé en ce qu'on utilise une séquence d'ADN qui code pour une protéine de type porcin ayant la séquence d'amino-acides :

1                                                                    15
IleValGlyGlyArgArgAlaGlnProGlnGluPheProPheLeu

                                                                     30
AlaSerIleGlnLysGlnGlyArgProPheCysAlaGlyAlaLeu

                                                                     45
ValHisProArgPheValLeuThrAlaAlaSerCysPheArgGly

                                                                     60
LysAsnSerGlySerAlaSerValValLeuGlyAlaTyrAspLeu

                                                                     75
ArgGlnGlnGluGlnSerArgGlnThrPheSerIleArgSerIle

                                                                     90
SerGlnAsnGlyTyrAspProArgGlnAsnLeuAsnAspValLeu

                                                                     105
LeuLeuGlnLeuAspArgGluAlaArgLeuThrProSerValAla

                                                                     120
LeuValProLeuProProGlnAsnAlaThrValGluAlaGlyThr

                                                                     135
AsnCysGlnValAlaGlyTrpGlyThrGlnArgLeuArgArgLeu

                                                                     150
PheSerArgPheProArgValLeuArgValThrValThrSerAsn

                                                                     165

```
                ProCysLeuProArgAspMetCysIleGlyValPheSerArgArg

                                                            180
                GlyArgIleSerGlnGlyAspArgGlyThrProLeuValCysAsn


                                                            195
                GlyLeuAlaGlnGlyValAlaSerPheLeuArgArgArgPheXxx

                196                                         210
                XxxSerSerGlyPhePheThrArgValAlaLeuPheArgAsnTrp


                                     217
                IleAspSerValLeuAsnXxx
```

dans laquelle X195 et X196 représentent des amino-acides arbitraires, et X217 représente un ou deux amino-acides arbitraires.

**7.** Procédé suivant la revendication 5, caractérisé en ce qu'on utilise une séquence d'ADN qui code pour une protéine de type humain ayant la séquence d'amino-acides :
à partir de l'extrémité N-terminale

```
                1                                            15
                IleValGlyGlyArgLysAlaArgProArgGlnPheProPheLeu


                                                             30
                AlaSerIleGlnAsnGlnGlyArgHisPheCysGlyGlyAlaLeu


                                35
                IleHisAlaArgPhe
```

à partir de l'extrémité C-terminale
```
                n-15                                    n-1   n

                ValAlaLeuPheArgAspTrpIleAspGlyValLeuAsnAsnProGly
```

n désignant le nombre total d'amino-acides dans la séquence de la protéine.

**8.** Procédé suivant la revendication 7, caractérisé en ce que la séquence d'ADN utilisée code pour une protéine ayant la séquence d'amino-acides suivante :
à partir de l'extrémité N-terminale

1            15

IleValGlyGlyArgLysAlaArgProArgGlnPheProPheLeu

               30

AlaSerIleGlnAsnGlnGlyArgHisPheCysGlyGlyAlaLeu

               45

IleHisAlaArgPheValMetThrAlaAlaSerCysPheGlnSer

               60

GlnAsnProGlyValSerThrValValLeuGlyAlaTyrAspLeu

               73

ArgArgArgGluArgGlnSerArgGlnThrPheSerIle

à partir de l'extrémité C-terminale

n-29           n-15

SerLeuGlyProCysGlyArgGlyProAspPhePheThrArgVal

               n

AlaLeuPheArgAspTrpIleAspGlyValLeuAsnAsnProGly

n répondant à la définition suivant la revendication 5.

9. Procédé suivant la revendication 7, caractérisé en ce que la séquence d'ADN utilisée code pour une protéine ayant la séquence d'amino-acides suivante :
à partir de l'extrémité N-terminale

1                                                              15
IleValGlyGlyArgLysAlaArgProArgGlnPheProPheLeu

                                                               30
AlaSerIleGlnAsnGlnGlyArgHisPheCysGlyGlyAlaLeu

                                                               45
IleHisAlaArgPheValMetThrAlaAlaSerCysPheGlnSer

                                                               60
GlnAsnProGlyValSerThrValValLeuGlyAlaTyrAspLeu

                                                       73
ArgArgArgGluArgGlnSerArgGlnThrPheSerIle


69
GlnThrPheSerIleUuuUuuMetSerGluAsnGlyTyrAspPro

GlnGln
- - - - - -


LeuGlnLeuAspArgGluAlaXxxLeuThrSerXxxValThrIle

LeuProLeuPro
- - - - - - - - - - - -


GluAlaGlyThrArgCysGlnValAlaGlyTrpGlySerGlnArg
- - - - - - - - - - - - - - - - - - -


LeuSerArgPheProArg

----------------

PheValXxxValThrValThrProGluAspGlnCysArgProAsn

AsnValCysThrGlyValLeuThrArg

----------------------------

UuuGlyGlyIleCysAsnGlyAspGlyUuuThrProValLeu

------------------------------------------

dans laquelle Uuu représente un amino-acide inconnu et Xxx représente un site de glycosylation possible, probablement Asn ;

à partir de l'extrémité C-terminale

n-29                                                    n-15

SerLeuGlyProCysGlyArgGlyProAspPhePheThrArgVal

n

AlaLeuPheArgAspTrpIleAspGlyValLeuAsnAsnProGly

dans laquelle n répond à la définition suivant la revendication 5.

**10.** Procédé suivant la revendication 7, caractérisé en ce que la séquence d'ADN utilisée code pour une protéine ayant la séquence d'amino-acides suivante :

à partir de l'extrémité N-terminale

1                                                      15

IleValGlyGlyArgLysAlaArgProArgGlnPheProPheLeu

30

AlaSerIleGlnAsnGlnGlyArgHisPheCysGlyGlyAlaLeu

45
IleHisAlaArgPheValMetThrAlaAlaSerCysPheGlnSer

60
GlnAsnProGlyValSerThrValValLeuGlyAlaTyrAspLeu

75
ArgArgArgGluArgGlnSerArgGlnThrPheSerIleUuuUuu

85
MetSerGluAsnGlyTyrAspProGlnGln(..............

.....)LeuGlnLeuAspArgGluAlaXxxLeuThrSerXxxVal

ThrIleLeuProLeuPro(................)GluAlaGly

ThrArgCysGlnValAlaGlyTrpGlySerGlnArg(........

..)LeuSerArgPheProArgPheValXxxValThrValThrPro

GluAspGlnCysArgProAsnAsnValCysThrGlyValLeuThr

ArgUuuGlyGlyIleCysAsnGlyAspGlyUuuThrProValLeu

n-29
(...........................)SerLeuGlyProCys

GlyArgGlyProAspPhePheThrArgValAlaLeuPheArgAsp

n

TrpIleAspGlyValLeuAsnAsnProGly

dans laquelle n répond à la définition suivant la revendication 5 ; Uuu et Xxx répondent aux définitions suivant la revendication 7.

FIG.1

FIG.2

FIG. 3

FIG. 4

FIG. 5

FIG. 6